# EUROPEAN PATENT APPLICATION

(11) **EP 4 130 228 A2**
(43) Date of publication of application: **08.02.2023**
(21) Application number: 22188408.3
(22) Date of filing: 02.08.2022
(51) Int. Cl.: C12M 1/00

(54) **SPERM SORTING DEVICE AND METHOD**

(30) Priority: 02.08.2021 TW 110128419; 21.03.2022 TW 111110406; 27.07.2022 US 202217815484
(71) Applicant: Bonraybio Co., Ltd., Taichung City 41280 (TW)
(72) Inventor: HSU, Cheng-Teng, 41280 Taichung City (TW); HSU, George Chengkang, 41280 Taichung City (TW); GAO, Han-Yuan, 41280 Taichung City (TW)
(74) Representative: Keltie LLP

(57) **Abstract**

A sperm sorting device suitable for selectively isolating motile sperms from a semen specimen includes a base and a sorting unit. The base comprises a central protruding block including a truncated cone-shaped surface, a bottom wall that surrounds the central protruding block, and an intermediate ring. The intermediate ring connects to the bottom wall via an inner wall surface. The sorting unit includes a sorting ring and a protruding ring. The sorting ring includes a central hole for a sorting filter that filters the semen specimen and is beveled at an angle that increases a permeable surface of the sorting filter. The protruding ring connects to the sorting ring via an inner sleeve surface. The sperm sorting device enhances sperm sorting quality by allowing the motile sperms to swim upward through the first sorting filter naturally.

## Description

### TECHNICAL FIELD

The present disclosure relates to a sperm sorting device and method.

### BACKGROUND

Sperm sorting systems are commonly used to collect motile sperm for in vitro fertilization. A sperm sorting system may include, among other components, inlets, outlets, filters, chambers, housings, and flow paths. An operator may insert a semen specimen into the sperm sorting system, which may isolate motile sperm from the semen specimen such that the operator may collect the motile sperm.

However, conventional sperm sorting systems have a few drawbacks. First, conventional sperm sporting systems are difficult to manufacture cheaply but with high quantity. Second, the structure of conventional sperm sorting device may affect sperm sorting quality in that trapped air may push sperms with low motility through its filter. Consequently, not all the sperms collected from the top chamber are highly motile, and the sperm sorting quality is low. Another drawback is that paths between components of the sperm sorting system may be long and include turns, so a semen sample must be injected via a syringe in order to generate the amount of pushing pressure required to drive the semen sample into a bottom chamber. The use of a syringe, however, causes inconvenience of operation. A further drawback is that that the entire sperm sorting system must be taken to a microscope for analysis, which is also an inconvenience of operation. Thus, a system that mitigates such drawbacks for sperm sorting is necessary.

### SUMMARY

The present disclosure provides a sperm sorting device suitable for selectively isolating motile sperms (e.g., sperms from the semen specimen with forward progressions over a threshold amount, such as 25 micrometers per second) from a semen specimen. The sperm sorting device includes a base and a sorting unit.

The base includes a central protruding block, a bottom wall, an intermediate ring, and a surrounding wall. the central protruding block includes a truncated cone-shaped surface. The bottom wall surrounds the central protruding block. The intermediate ring connects to an external edge of the bottom wall via a first inner wall surface. The surrounding wall extends from the periphery of the bottom wall toward the top end of the base and surrounds an axis. The surrounding wall is cylindrical and has a top surface and a second inner wall surface. The surrounding wall connects inward to the intermediate ring via the second inner wall surface. The bottom wall and the surrounding wall jointly define a receiving space that has an opening.

The sorting unit includes a first sorter configured to be positioned on the base. The first sorter has a first mounting member and a first sorting filter. The first mounting member has a first protruding ring and a first sleeve, and the first sleeve surrounds the axis and is configured to be placed in the receiving space. The first sleeve has a first inner sleeve surface connected to an outer portion of a sorting ring and an inner portion of a first protruding ring and a first outer sleeve surface surrounding the first inner sleeve surface. The protruding ring is on the periphery of the sorting unit as part of the first mounting member. The protruding ring is structurally adapted to sit on top of the base. The sorting ring is situated at the bottom of the sorting unit and includes a central hole for the first sorting filter to spans and filters the semen specimen. A side of the sorting ring facing the sorting filter is beveled at an angle that increases a permeable surface of the sorting filter. The first sorting filter, the bottom wall, and the inner wall surface of the surrounding wall jointly define a first specimen chamber for receiving the semen specimen.

In some embodiments, the sorting ring includes a position-limiting groove that can receive an end of a liquid taking device. The position-limiting groove is indented below a top plane of the sorting ring and may be open to the central hole. The position-limiting groove extends inward towards the central hole from a bottom plane of the sorting ring to a top plane of the sorting ring. The sorting ring may include a set of vent holes, one on each side of the position-limiting groove, to allow air to escape from the base during sorting. In further embodiments, the sperm sorting device includes a second sorter configured to sit on top of the first sorter. In these embodiments, the first sorter includes the set of vent holes and the second sorter includes the position-limiting groove.

The sperm sorting device of the present disclosure has the following effects: Air that exists in the first specimen chamber in the first place can be squeezed out of the first specimen chamber so that highly motile sperms in a semen specimen can swim upward through the first sorting filter naturally, and sperm sorting quality is thus enhanced.

The present disclosure further provides a sperm sorting method that includes the following steps:
(A) The foregoing sperm sorting device is prepared;
(B) A semen specimen is provided;
(C) The semen specimen is added, in drops, into the receiving space through the opening of the base;
(D) The first sleeve of the first sorter is placed into and positioned in the receiving space, with the first sorting filter located above the semen specimen, the semen specimen received in the first specimen chamber, and the air in the first specimen chamber squeezed out of the first specimen chamber;
(E) A first sorting medium is applied, in drops, onto the first sorting filter;
(F) The sperm sorting device is set aside for 3 minutes to 60 minutes, during which time a plurality of motile sperms in the semen specimen that have high motility will swim upward through the first micropores of the first sorting filter into a first sorting chamber and be mixed with the first sorting medium; and
(G) The first sorting medium on the first sorting filter is collected along with the motile sperms having high motility.

The sperm sorting method of the present disclosure has the following effects: air that exists in the first specimen chamber in the first place can be squeezed out of the first specimen chamber so that highly motile sperms in a semen specimen can swim upward through the first sorting filter naturally, thus enhancing sperm sorting quality compared to conventional systems.

### BRIEF DESCRIPTION OF THE SEVERAL VIEWS OF THE DRAWINGS

Other features and effects of the present disclosure can be better understood by referring to the following detailed description in conjunction with the accompanying drawings, in which:
FIG. 1 is an assembled sectional view of the system disclosed in US Patent No. 10,422,737 B2;
FIG. 2 is an assembled perspective view of a sperm sorting device according to a first embodiment of the disclosure;
FIG. 3 is an exploded perspective view of the first embodiment with a base and a sorting unit that includes a first sorter;
FIG. 4 is an assembled sectional view of the first embodiment;
FIG. 5 shows a state of use of the first embodiment;
FIG. 6 is an assembled sectional view of the first embodiment including an annular groove in the base and aa first annular groove in the first sorter;
FIG. 7 is an assembled perspective view of the sperm sorting device according to a second embodiment of the disclosure, which includes a base, cover, and sorting unit with two sorters;
FIG. 8 is an exploded perspective view of the second embodiment;
FIG. 9 is an assembled sectional view of the second embodiment;
FIG. 10 shows a state of use of the second embodiment where the cover is turned upside down to hold a sorter;
FIG. 11 is an exploded perspective view of the sperm sorting device according to a third embodiment of the disclosure, which includes a base, a cover, and a sorting unit with a first sorter and a second sorter;
FIG. 12 is an assembled sectional view of the third embodiment;
FIG. 13 shows a state of use of the third embodiment;
FIG. 14 is an assembled perspective view of the sperm sorting device according to a fourth embodiment of the disclosure;
FIG. 15 is an exploded perspective view of the fourth including a second sorter in the sorting unit;
FIG. 16 is an assembled sectional view of the fourth embodiment;
FIG. 17 is an exploded perspective view of the sperm sorting device according to a fifth embodiment of the disclosure, which includes a base, a cover, and a sorting unit with two second sorters;
FIG. 18 is an assembled sectional view of the fifth embodiment;
FIG. 19 shows a state of use of the fifth embodiment where the cover is turned upside down to hold one of the second sorters;
FIG. 20 is an assembled perspective view of the sperm sorting device according to a sixth embodiment of the disclosure, which includes a base, the sorting with a first sorter, and a height adjustment unit;
FIG. 21 is an exploded perspective view of the sixth embodiment;
FIG. 22 is a sectional view of the sixth embodiment based on FIG. 20;
FIG. 23 shows a first mounting member of the sixth embodiment rotated 60° with respect to the base;
FIG. 24 is a sectional view of the sixth embodiment based on FIG. 23;
FIG. 25 shows the first mounting member of the sixth embodiment rotated through 120° with respect to the base;
FIG. 26 is a sectional view of the sixth embodiment based on FIG. 25;
FIG. 27 is an exploded perspective view of the sperm sorting device according to a seventh embodiment of the disclosure, which includes a base, a sorting unit with a first sorter, and a height adjustment unit;
FIG. 28 is an assembled sectional view of the seventh embodiment;
FIG. 29 is an exploded perspective view of the sperm sorting device according to an eighth embodiment of the disclosure, which includes a base, a sorting unit with two first sorters, and a height adjustment unit;
FIG. 30 is an assembled sectional view of the eighth embodiment;
FIG. 31 is an assembled sectional view of the sperm sorting device according to a ninth embodiment of the disclosure;
FIG. 32 is an assembled sectional view of the sperm sorting device according to a tenth embodiment of the disclosure;
FIG. 33 is an assembled sectional view of the sperm sorting device according to an eleventh embodiment of the disclosure;
FIG. 34 shows statistical charts that provide a comparison of total motility and forward motility between motile sperms obtained according to the disclosure, sperms obtained from the source semen specimen, and sperms obtained with a prior art device;
FIG. 35 is a partially exploded perspective view of the sperm sorting device according to a twelfth embodiment of the disclosure;
FIG. 36 is an exploded perspective view of the twelfth embodiment, which includes a base, a sorting unit, and an interlocking unit;
FIG. 37 is another exploded perspective view of the twelfth embodiment from a different viewing angle from that of FIG. 36;
FIG. 38 is an assembled sectional view of the twelfth embodiment, showing a state of use;
FIG. 39 is a partially exploded perspective view of the sperm sorting device according to a thirteenth embodiment of the disclosure;
FIG. 40 is an exploded perspective view of the thirteenth embodiment, which includes the base from the twelfth embodiment, the sorting unit from the first embodiment, the interlocking unit from the twelfth embodiment, and an auxiliary frame for a liquid taking device;
FIG. 41 is an assembled sectional view of the thirteenth embodiment, showing a state of use;
FIG. 42 is a partially exploded perspective view of the sperm sorting device according to a fourteenth embodiment of the disclosure;
FIG. 43 is an exploded perspective view of the fourteenth embodiment, which includes the base from the twelfth embodiment and a sorting unit with a third sorter;
FIG. 44 is an assembled sectional view of the fourteenth embodiment, showing a state of use;
FIG. 45 is a partially exploded perspective view of the sperm sorting device according to a fifteenth embodiment of the disclosure;
FIG. 46 is an exploded perspective view of the fifteenth embodiment, which includes the base from the twelfth embodiment and the sorting unit from the fourteenth embodiment;
FIG. 47 is an assembled sectional view of the fifteenth embodiment, showing a state of use;
FIG. 48 is a partially exploded perspective view of the sperm sorting device according to a sixteenth embodiment of the disclosure;
FIG. 49 is an exploded perspective view of the sixteenth embodiment, which includes the base from the twelfth embodiment and the sorting unit from the fourteenth embodiment;
FIG. 50 is an assembled sectional view of the sixteenth embodiment, showing a state of use;
FIG. 51 is a partially exploded perspective view of the sperm sorting device according to a seventeenth embodiment of the disclosure;
FIG. 52 is an exploded perspective view of the seventeenth embodiment, which includes the base from the twelfth embodiment and a sorting unit with a fourth sorter and a fifth sorter; and
FIG. 53 is an assembled sectional view of the seventeenth embodiment, showing a state of use.

### DETAILED DESCRIPTION

### Description of Related Art

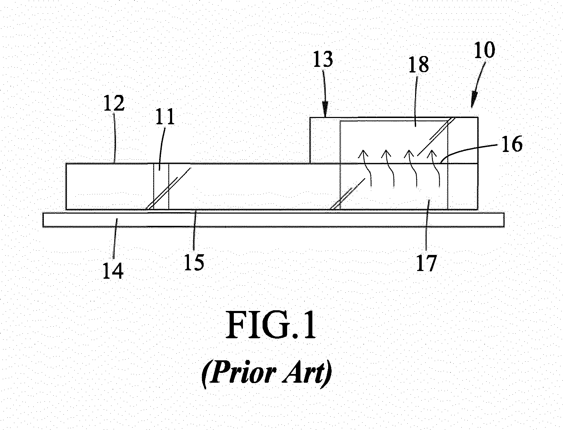

Referring to FIG. 1, the sperm sorting system 10 disclosed in US Patent No. 10,422,737 B2 includes a housing 12 formed with an inlet 11, a collection chamber 13 provided in the housing 12 and located beside the inlet 11, a microfluidic chip 14 mounted on the bottom side of the housing 12, a flow path 15 extending from the inlet 11 to the collection chamber 13, and a filter 16 provided in the collection chamber 13. The collection chamber 13 includes a bottom chamber 17 adjacent to the microfluidic chip 14 and a top chamber 18 located on top of the bottom chamber 17 and farther away from the microfluidic chip 14 than is the bottom chamber 17. The bottom chamber 17 is configured to collect the semen sample injected through the inlet 11 into the flow path 15 so that motile sperms in the semen sample can swim upward through the filter 16 into the top chamber 18 and thus become selectively isolated from the semen sample.

While the sperm sorting system 10 can sort sperms, it has a few drawbacks. First, the flow path 15 and the bottom chamber 17 are full of air in the first place, but the micropores in the filter 16 have such small diameters that, although sperms can pass through the micropores, air cannot do so with ease. When a semen sample is injected through the inlet 11 into the flow path 15 and enters the bottom chamber 17, therefore, the pushing pressure generated by the injection will compress the air in the bottom chamber 17, and the semen sample that has entered the bottom chamber 17 will be driven toward the top chamber 18 by the same pressure such that sperms with low motility are pushed through the filter 16 into the top chamber 18. Consequently, not all the sperms collected from the top chamber 18 are highly motile, and the sperm sorting quality is low.

Second, the path from the inlet 11 to the bottom chamber 17 is relatively long and has two turns, so a semen sample must be injected through the inlet 11 into the flow path 15 by a syringe in order to generate the pushing pressure required to drive the semen sample into the bottom chamber 17. The use of a syringe, however, causes inconvenience of operation. Further, the path is hard and expensive to manufacture and the turns are unnecessarily restrictive for the flow of air.

Third, when a sorting operation is completed, and it is desired to analyze the sperms obtained, the entire sperm sorting system 10 must be taken to a microscope for analysis, and this causes inconvenience of operation, too.

The following description uses relative terms of position, such as "top" and "bottom", in reference to the directions shown in the drawings and directions associated with normal use, and that similar elements are denoted by the same reference numeral. In addition, while elements referred to herein as "sleeve" or "ring" are shown in the drawings as circular, these elements may not be circular in some embodiments.

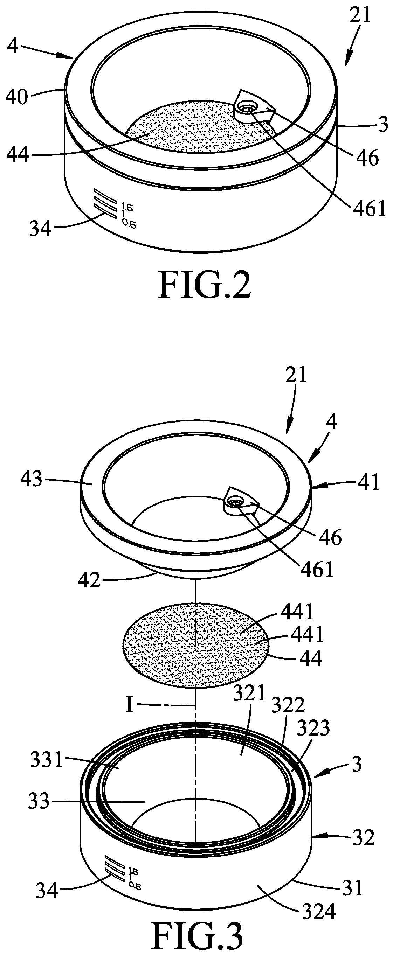

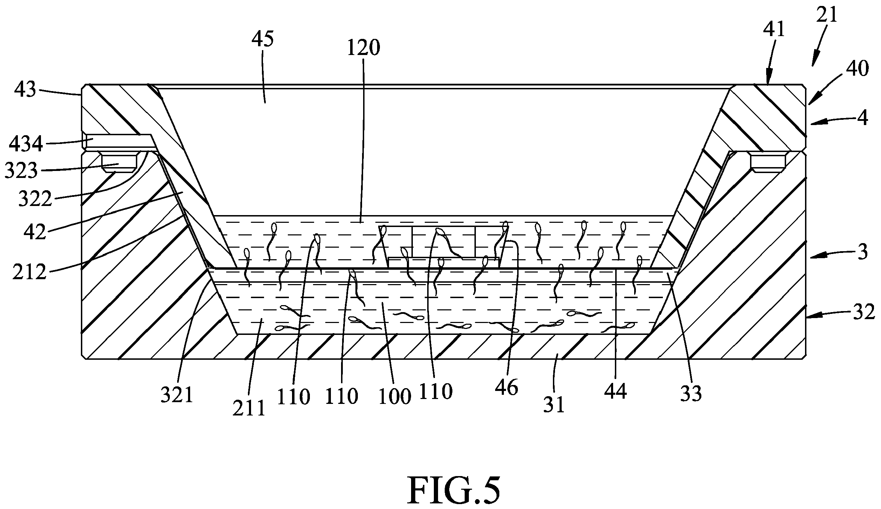

Referring to FIGS. 2-5, the sperm sorting device 21 (also referred to as a sperm separation device herein) according to the first embodiment of the present disclosure is suitable for selectively isolating a plurality of motile sperms 110 from a semen specimen 100 and includes a base 3 and a sorting unit 4.

The base 3 includes a bottom wall 31 and a surrounding wall 32. The surrounding wall 32 extends from the periphery of the bottom wall 31 toward the top end of the base 3 and surrounds an axis I. The bottom wall 31 and the surrounding wall 32 jointly define a receiving space 33 that has an opening 331. The surrounding wall 32 has a inner wall surface 321 and a top surface 322. The top surface 322 is formed with an overflow channel 323. In this embodiment, the inner wall surface 321 is an annular inclined surface that extends, and whose diameter is gradually increased, from the bottom wall 31 toward the top surface 322 along the axis I, and the receiving space 33 is a truncated cone-shaped space with a wider top than the bottom.

It is worth noting that the surrounding wall 32 further has an outer wall surface 324 surrounding the inner wall surface 321, and that the base 3 further includes indicating graduation marks 34 provided on the outer wall surface 324.

The sorting unit 4 includes a first sorter 40, and the first sorter 40 has a first mounting member 41 and a first sorting filter 44. The first mounting member 41 has a first sleeve 42 and a first protruding ring 43. The first sleeve 42 surrounds the axis I and is configured to be placed in the receiving space 33. The first protruding ring 43 extends from the top end of the first sleeve 42 in a direction away from the axis I and is configured to be disposed on the top surface 322 of the surrounding wall 32.

The first sleeve 42 has a first inner sleeve surface 421, a first outer sleeve surface 422 surrounding the first inner sleeve surface 421, and a first bottom sleeve surface 423 connecting the first inner sleeve surface 421 and the first outer sleeve surface 422. In this embodiment, the diameter of the first sleeve 42 is gradually increased as the first sleeve 42 extends from its bottom end toward its top end along the axis I, meaning the first sleeve 42 is a truncated cone-shaped sleeve with a wider top than the bottom.

The first protruding ring 43 has an annular top surface 431, an annular bottom surface 432, and an annular peripheral surface 433 connecting the annular top surface 431 and the annular bottom surface 432. The annular bottom surface 432 forms a first air escape groove 434 that extends from the first outer sleeve surface 422 to the annular peripheral surface 433.

The first sorting filter 44 is fixed on the first bottom sleeve surface 423 of the first sleeve 42. The first sorting filter 44 and the first inner sleeve surface 421 jointly define a first sorting chamber 45 that has an open top end. The first sorting filter 44 has a plurality of first micropores 441 through which the motile sperms 110 may to pass. The material of the first sorting filter 44 may be a polycarbonate, polystyrene, polyethylene terephthalate, polymethyl methacrylate, polypropylene, polytetrafluoroethylene, nylon, a cellulose ester, a composite material, engineered plastic, or the like. The area of the first sorting filter 44 is smaller than 180 cm2, and the pore diameter of each first micropore 441 ranges from 2 µm to 50 µm. The first micropores 441 provide the first sorting filter 44 with a porosity ranging from 5% to 90%. In this embodiment, the pore diameters range from 8 µm to 20 µm, and the porosity ranges from 5% to 20%.

The first sorting filter 44 may be fixed to the first bottom sleeve surface 423 of the first sleeve 42 by, for example, ultrasonic welding, a common heat fusion method, infrared welding, or a UV-curing adhesive. The first sorting filter 44 may, in alternative embodiments, be fixed to the first inner sleeve surface 421 of the first sleeve 42 or to an inner ring that extends, and whose diameter is gradually reduced, from the bottom end of the first sleeve 42 toward the axis I.

The first sorting filter 44 has a fixed portion 442 and a permeable portion 443. The fixed portion 442 surrounds the axis I and is fixed on the first sleeve 42. The permeable portion 443 extends from the fixed portion 442 toward the axis I in an unlevel manner. The permeable portion 443 may be convex upward, concave downward, or wavy so as to increase the permeable area of the permeable portion 443.

Once the first sleeve 42 of the first mounting member 41 is placed in the receiving space 33, the first sorting filter 44, the bottom wall 31, and the inner wall surface 321 of the surrounding wall 32 jointly define a first specimen chamber 211 for receiving the semen specimen 100, and the inner wall surface 321 of the surrounding wall 32 and the first outer sleeve surface 422 of the first sleeve 42 jointly define a first passage gap 212 in communication with the first specimen chamber 211 and the first air escape groove 434.

The volume of the first specimen chamber 211 may range from 12 µl to 400 ml, the distance between the first sorting filter 44 and the bottom wall 31 may range from 50 µm to 8.5 cm, the first passage gap 212 may be smaller than 3 mm, and the first passage gap 212 has a bottom area parallel to the bottom wall 31 and may range from 0.25 mm² to 600 mm².

The first sorter 40 further includes a liquid-taking-device socket 46 provided on the first inner sleeve surface 421 of the first mounting member 41 and located in the first sorting chamber 45. The liquid-taking-device socket 46 has an insertion hole 461 where a liquid taking device (e.g., a syringe, not shown) can be inserted and positioned to extract sperm sorted from the semen specimen. The insertion hole has a hole diameter ranging from 4 µm to 5 mm.

The sperm sorting device 21 may be used in a sperm sorting method to separate motile sperms from a semen specimen. The sperm sorting method may include:
Step 1: preparing the sperm sorting device 21;
Step 2: providing the semen specimen 100 with a volume of about 0.5 cc to 50 cc;
Step 3: adding the semen specimen 100, in drops, into the receiving space 33 through the opening 331 of the base 3;
Step 4: placing the first sleeve 42 of the first sorter 40 into, and positioning the first sleeve 42 in, the receiving space 33, with the first sorting filter 44 located above the semen specimen 100, the semen specimen 100 received in the first specimen chamber 211, and the air in the first specimen chamber 211 squeezed out of the first specimen chamber 211 or squeezed into the first passage gap 212. The semen specimen 100 may take up at least 80% of the volume of the first specimen chamber 211 so that the motile sperms 110 can pass through the first sorting filter 44 successfully;
Step 5: applying a first sorting medium 120 (i.e., sperm washing medium, about 5 cc to 50 cc) onto the first sorting filter 44 in drops so that a portion of the first sorting medium 120 seeps into the first specimen chamber 211 and thereby forms a continuous liquid surface. The first sorting medium 120 may be any diluting liquid suitable for the processing of sperms;
Step 6: putting the sperm sorting device 21 aside for 3 minutes to 60 minutes, during which time the motile sperms 110 in the semen specimen 100 that have high motility will swim upward for a maximum distance of about 15 mm, pass through the first micropores 441 of the first sorting filter 44, enter the first sorting chamber 45, and be mixed with the first sorting medium 120; and
Step 7: collecting, via a liquid taking device, the first sorting medium 120 on the first sorting filter 44 along with the motile sperms 110 that have high motility.

Through the foregoing steps, the motile sperms 110 with high motility can be separated from the semen specimen. The present disclosure is so designed that the semen specimen 100 is added, in drops, into the receiving space 33. The air in the first specimen chamber 211 can be squeezed out of the first specimen chamber 211 or squeezed into the first passage gap 212 while the first sleeve 42 of the first mounting member 41 is being placed into the receiving space 33. The air can further be discharged to the outside through the first air escape groove 434 when exceeding the volume of the first passage gap 212, lest the air pressure in the first specimen chamber 211 be affected, the objective being to allow the motile sperms 110 in the semen specimen 100 that have high motility to swim upward through the first sorting filter 44 and enter the first sorting chamber 45 naturally. The disclosure therefore ensures that all the motile sperms 110 collected from the first sorting filter 44 have high motility, and sperm sorting quality is thus enhanced.

The following are worth noting:
(1) In the course in which an operator adds the semen specimen 100, in drops, into the receiving space 33, the volume of the semen specimen 100 added can be checked against the indicating graduation marks 34. If the operator inadvertently adds so much semen specimen 100 that the volume of the semen specimen 100 added exceeds the volume of the first specimen chamber 211, the excess semen specimen 100 can enter the overflow channel 323 of the base 3 through the first passage gap 212 so that the immediate surroundings will not be contaminated.
(2) To increase sorting efficiency, the first inner sleeve surface 421 of the first sleeve 42 may be coated with hyaluronic acid or progesterone, or additionally provided with an infrared lightbulb structure or a power coil structure. In some embodiments, the inner wall surface 321 of the surrounding wall 32 may be additionally provided with a bionic structure.
(3) In cases where the total swimming distance of the motile sperms 110 is less than 15 mm, a higher recovery rate of the motile sperms 110 can be achieved (i.e., more motile sperms 110 can be obtained) when the volume ratio of the first specimen chamber 211 to the first sorting chamber 45 is less than or equal to 1:1 than otherwise.
(4) With the liquid taking device inserted into and secured in the liquid-taking-device socket 46, the operator can keep the liquid taking device stead to achieve high operation efficiency.

Following step 6, the operator may take the first sorter 40 out of the base 3 and put the first sorter 40 directly on the stage of a microscope in order to analyze the motile sperms 110 obtained.

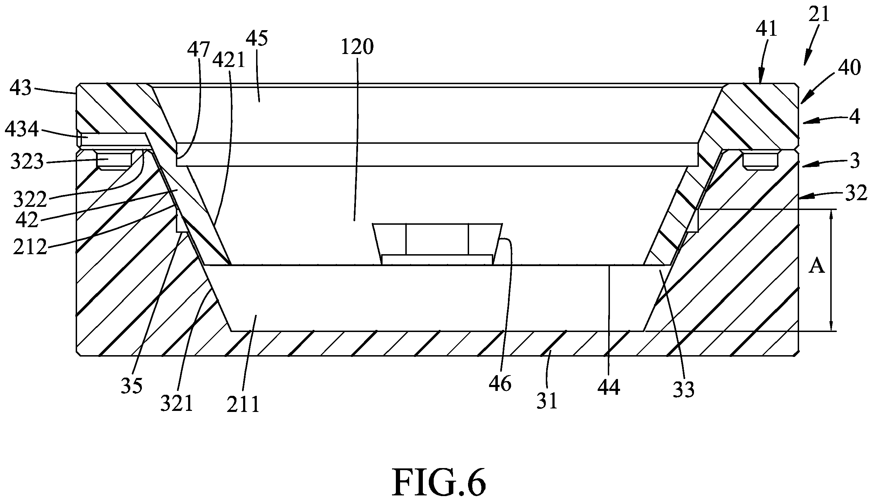

Referring to FIG. 6, the base 3 may further include an annular groove 35 provided in the inner wall surface 321 of the surrounding wall 32, and the first sorter 40 may further have a first annular groove 47 provided in the first inner sleeve surface 421 of the first sleeve 42. The annular groove 35 and the first annular groove 47 serve to prevent significant capillary action and block extended capillary action. The present disclosure has no limitation on the cross-sectional shape of each of the annular groove 35 and the first annular groove 47 or the height A at which each groove is provided.

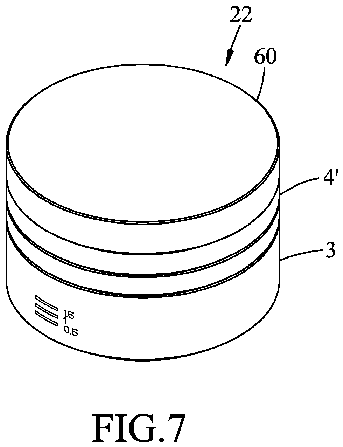

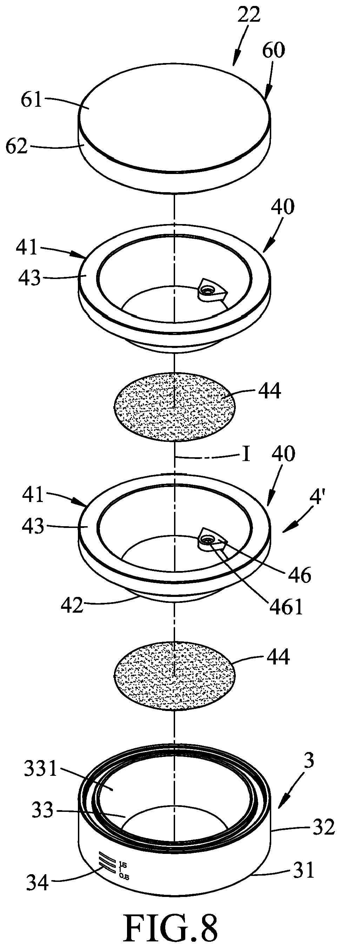

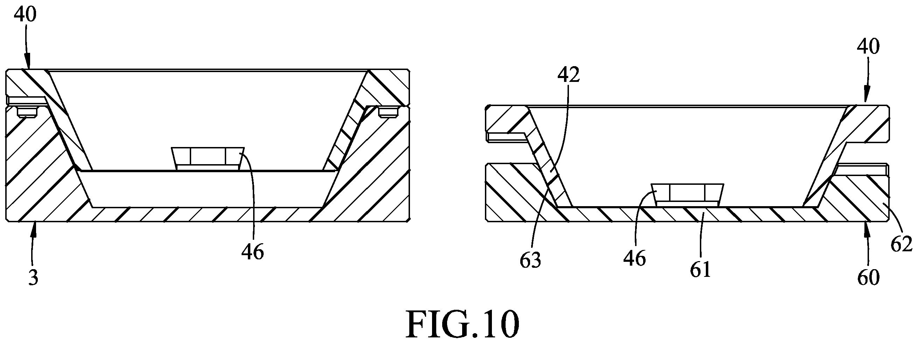

Referring to FIG. 7 to FIG. 10, the sperm sorting device 22 according to the second embodiment of the present disclosure includes the base 3, a sorting unit 4', and a cover 60.

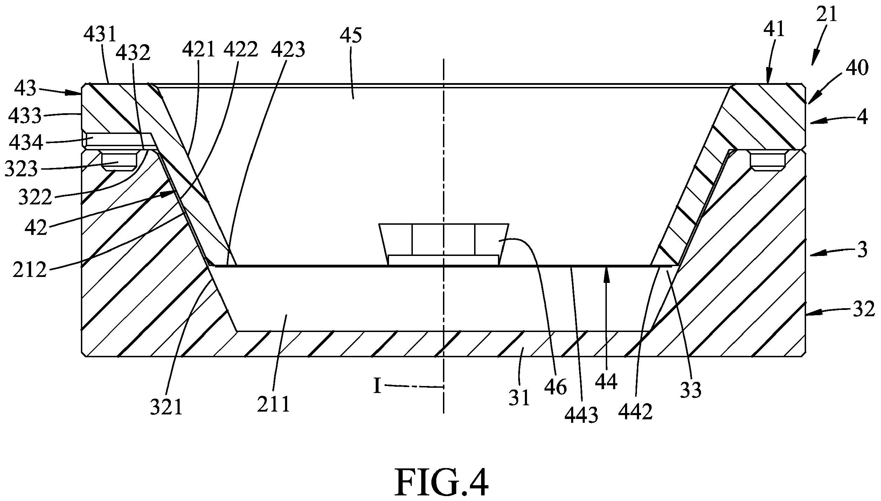

The sorting unit 4' includes two first sorters 40. One of the first sorters 40 (hereinafter referred to as the lower first sorter 40) is placed on the base 3 such that the lower first sorter 40 and the base 3 form the same structure as the sperm sorting device 21 (see FIG. 4). The first sleeve 42 of the other first sorter 40 (hereinafter referred to as the upper first sorter 40) is placed in the first sleeve 42 of the lower first sorter 40, with the first protruding ring 43 of the upper first sorter 40 located on the first protruding ring 43 of the lower first sorter 40 such that the upper first sorter 40 is superposed on the lower first sorter 40 along the axis I.

The cover 60 includes a top wall 61 and a top surrounding wall 62. The top surrounding wall 62 extends from the periphery of the top wall 61 toward the bottom end of the cover 60, surrounds the axis I, and is configured to be disposed on the first protruding ring 43 of the upper first sorter 40 (or of the lower first sorter 40 if the upper first sorter 40 is not used). The top wall 61 and the top surrounding wall 62 jointly define a top receiving space 63 that has an open bottom end.

When using the sperm sorting device 22, an operator may perform a one-tier or two-tier sperm sorting operation. The one-tier sperm sorting operation entails the structure assembled from the base 3 and one of the first sorters 40, whereas the two-tier sperm sorting operation entails the structure assembled from the base 3 and both first sorters 40 so that sperms with even higher motility can be obtained from the first sorting filter 44 of the upper first sorter 40. The two-tier sperm sorting operations are described in more detail below in connection with the third embodiment.

The cover 60 is so designed that either the top surrounding wall 62 can be placed on the first protruding ring 43 of the upper first sorter 40 (or of the lower first sorter 40 if the upper first sorter 40 is not used) to seal the first sorting chamber 45 of the upper (or lower) first sorter 40, or the cover 60 can be turned upside down, with the top receiving space 63 facing up so that the first sleeve 42 of either of the two first sorters 40 can be placed in the inverted top receiving space 63, thereby converting the cover 60 into a supporting platform.

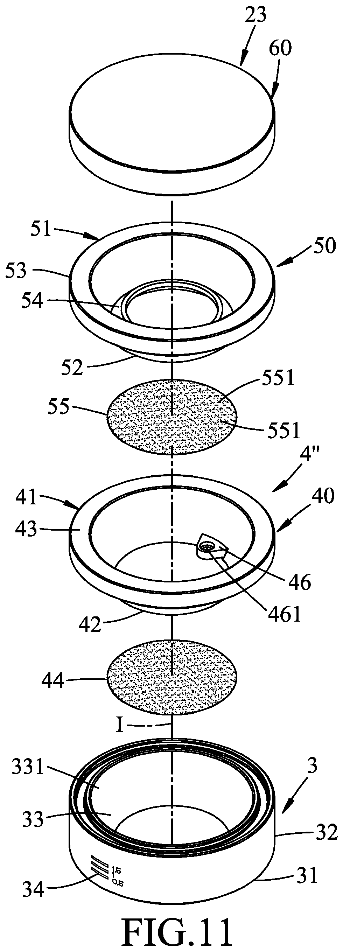

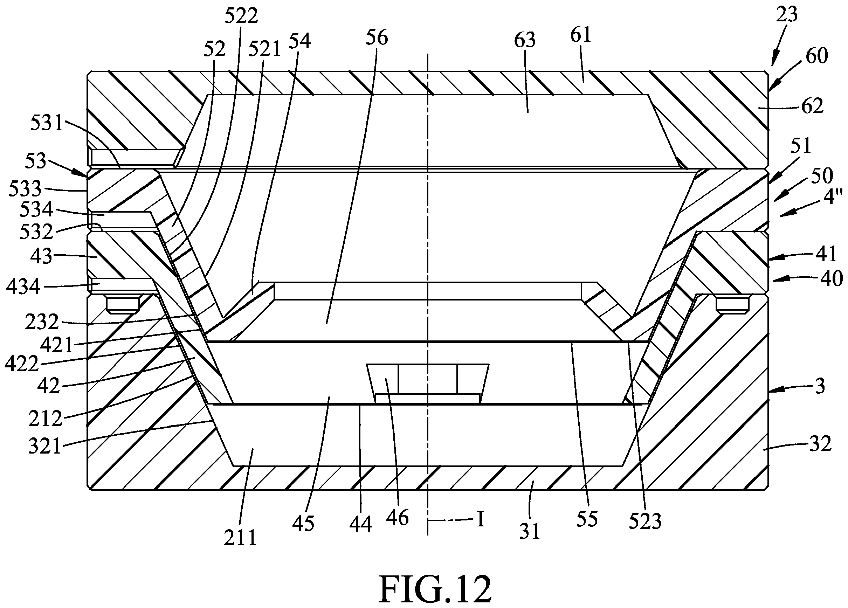

Referring to FIG. 11 to FIG. 13, the sperm sorting device 23 according to the third embodiment of the present disclosure includes the base 3, a sorting unit 4", and the cover 60. The sorting unit 4" includes one first sorter 40 and a second sorter 50. The second sorter 50 has a second mounting member 51 and a second sorting filter 55.

The second mounting member 51 has a second sleeve 52, a second protruding ring 53, and an inner ring 54. The second sleeve 52 surrounds the axis I and is configured to be placed in the first sleeve 42. The second protruding ring 53 extends from the top end of the second sleeve 52 in a direction away from the axis I and is configured to be disposed on the first protruding ring 43. The inner ring 54 extends from the bottom end of the second sleeve 52 toward the axis I and the top end of the second sleeve 52, and the diameter of the inner ring 54 is gradually reduced toward the top end of the second sleeve 52.

The second sleeve 52 has a second inner sleeve surface 521, a second outer sleeve surface 522 surrounding the second inner sleeve surface 521, and a second bottom sleeve surface 523 connecting the second inner sleeve surface 521 and the second outer sleeve surface 522. In this embodiment, the diameter of the second sleeve 52 is gradually increased as the second sleeve 52 extends from its bottom end toward its top end along the axis I, meaning the second sleeve 52 is a truncated cone-shaped sleeve with a wider top than bottom.

The second protruding ring 53 has an annular top surface 531, an annular bottom surface 532, and an annular peripheral surface 533 connecting the annular top surface 531 and the annular bottom surface 532. The annular bottom surface 532 forms a second air escape groove 534 that extends from the second outer sleeve surface 522 to the annular peripheral surface 533. The inner ring 54 is a truncated cone-shaped ring having a wider bottom than the top.

The second sorting filter 55 is fixed on the second bottom sleeve surface 523 of the second sleeve 52. The second sorting filter 55 and the inner ring 54 jointly define a second sorting chamber 56 that has an open top end. The second sorting filter 55 has a plurality of second micropores 551 through which a plurality of motile sperms 111 having higher motility than those motile sperms 110 able to pass through the first sorting filer 44 are allowed to pass. The material of the second sorting filter 55 may be the same as that of the first sorting filter 44 in some embodiments, or different than that of the first sorting filter 44 in other embodiments. The pore diameter of each second micropore 551 may range from 2 µm to 50 µm, and the second micropores 551 may provide the second sorting filter 55 with a porosity ranging from 5% to 90%. In this embodiment, the pore diameters of the second sorting filter 55 range from 8 µm to 12 µm, and the porosity of the second sorting filter 55 ranges from 5% to 20%. The area, material, pore diameters, and porosity of the second sorting filter 55 are not necessarily the same as those of the first sorting filter 44.

The second sorting chamber 56, which is jointly formed by the second sorting filter 55 and the truncated cone-shaped inner ring 54 having a wider bottom than the top, has a smaller volume than the first sorting chamber 45 so that the amount of the sorting medium to be used can be reduced, which lowers cost of use.

The cover 60 is so designed that either the top surrounding wall 62 can be placed on the second protruding ring 53 of the second sorter 50 to seal the second sorting chamber 56, or the cover 60 can be turned upside down, with the top receiving space 63 facing up so that the first sleeve 42 of the first sorter 40 or the second sleeve 52 of the second sorter 50 can be placed in the inverted top receiving space 63, thereby converting the cover 60 into a supporting platform.

When the first sleeve 42 of the first sorter 40 is placed in the receiving space 33, the first sorting filter 44, the bottom wall 31, and the inner wall surface 321 of the surrounding wall 32 jointly define the first specimen chamber 211 for receiving the semen specimen 100. The inner wall surface 321 of the surrounding wall 32 and the first outer sleeve surface 422 of the first sleeve 42 jointly define the first passage gap 212 in communication with the first specimen chamber 211 and the first air escape groove 434, where the first passage gap 212 may be 0.05 mm to 2 mm. Once the second sleeve 52 of the second sorter 50 is placed in the first sorting chamber 45 of the first sorter 40, the first inner sleeve surface 421 of the first sleeve 42 and the second outer sleeve surface 522 of the second sleeve 52 jointly define a second passage gap 232 that is in communication with the first sorting chamber 45 and the second air escape groove 534, and the second passage gap 232 may be 0.05 mm to 2 mm. The present disclosure further provides a sperm sorting method corresponding to the sperm sorting device 23, and the method may include the following steps:
Step 1: preparing the sperm sorting device 23;
Step 2: providing the semen specimen 100, whose volume is about 0.5 cc to 50 cc;
Step 3: adding the semen specimen 100, in drops, into the receiving space 33 through the opening 331 of the base 3;
Step 4: placing the first sleeve 42 of the first sorter 40 into, and positioning the first sleeve 42 in, the receiving space 33, with the first sorting filter 44 located above the semen specimen 100, the semen specimen 100 received in the first specimen chamber 211, and the air in the first specimen chamber 211 squeezed out of the first specimen chamber 211 or squeezed into the first passage gap 212. The semen specimen 100 may take up at least 80% of the volume of the first specimen chamber 211 so that the motile sperms 110 can pass through the first sorting filter 44 successfully;
Step 5: applying a first sorting medium 120 (i.e., sperm washing medium, about 5 cc to 50 cc) onto the first sorting filter 44 in drops so that a portion of the first sorting medium 120 seeps into the first specimen chamber 211 and thereby forms a continuous liquid surface, wherein the first sorting medium 120 may be any diluting liquid suitable for the processing of sperms;
Step 6: placing the second sleeve 52 of the second sorter 50 into the first sorting chamber 45 such that the second protruding ring 53 is located on the first protruding ring 43 while the second sorting filter 55 is located above the first sorting medium 120;
Step 7: applying a second sorting medium 121 (i.e., sperm washing medium, about 10 cc) onto the second sorting filter 55 in drops, wherein the second sorting medium 121 may be any diluting liquid suitable for the processing of sperms;
Step 8: putting the sperm sorting device 23 aside (e.g., for 3 minutes to 60 minutes), during which time the motile sperms 110 in the semen specimen 100 that have high motility may swim upward for a maximum distance of about 15 mm, then pass through the first micropores 441 of the first sorting filter 44, enter the first sorting chamber 45, and be mixed with the first sorting medium 120, and a plurality of motile sperms 111 on the first sorting filter 44 that have even higher motility may swim upward through the second micropores 551 of the second sorting filter 55 into the second sorting chamber 56 and be mixed with the second sorting medium 121; and
Step 9: collecting the second sorting medium 121 on the second sorting filter 55 along with the motile sperms 111, which have higher motility than those motile sperms 110 able to pass through only one sorting filter.

The foregoing steps allow the motile sperms 111, which have higher motility than those motile sperms 110 able to pass through only one sorting filter, to be obtained, and the sperm sorting quality of the present disclosure is thus enhanced.

In some embodiments, the sperm sorting device 23 may form an alternative structure by first placing the second sleeve 52 of the second sorter 50 into the receiving space 33 of the base 3 and then placing the first sleeve 42 of the first sorter 40 into the second sleeve 52 of the second sorter 50. This alternative structure allows the motile sperms 111, which have higher motility than those motile sperms 110 able to pass through only one sorting filter, to be obtained. The first sorting medium 120 and the second sorting medium 121 may be different sorting mediums.

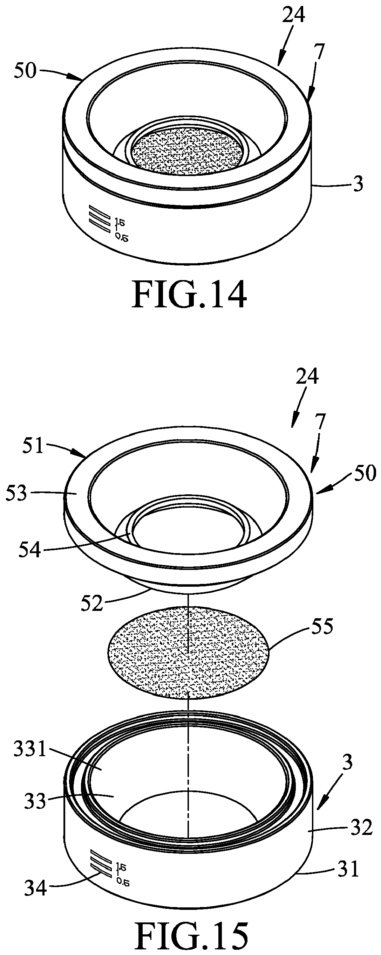

Referring to FIG. 14 to FIG. 16, the sperm sorting device 24 according to the fourth embodiment of the present disclosure includes the base 3 and a sorting unit 7. The sorting unit 7 includes the second sorter 50.

When the second sleeve 52 of the second sorter 50 is placed in the receiving space 33, the second sorting filter 55, the bottom wall 31, and the inner wall surface 321 of the surrounding wall 32 jointly define a second specimen chamber 241 for receiving the semen specimen 100, and the inner wall surface 321 of the surrounding wall 32 and the second outer sleeve surface 522 of the second sleeve 52 jointly define a third passage gap 242 that is in communication with the second specimen chamber 241 and the second air escape groove 534. The third passage gap 242 may be 0.05 mm to 2 mm.

The sperm sorting device 24 has the same function as the sperm sorting device 21 (see FIG. 4) but is different from the sperm sorting device 21 in that the second sorting chamber 56 that has a smaller volume than the first sorting chamber 45. The sperm sorting device 24, therefore, requires a smaller amount of sorting medium and incurs a lower cost of use than the sperm sorting device 21.

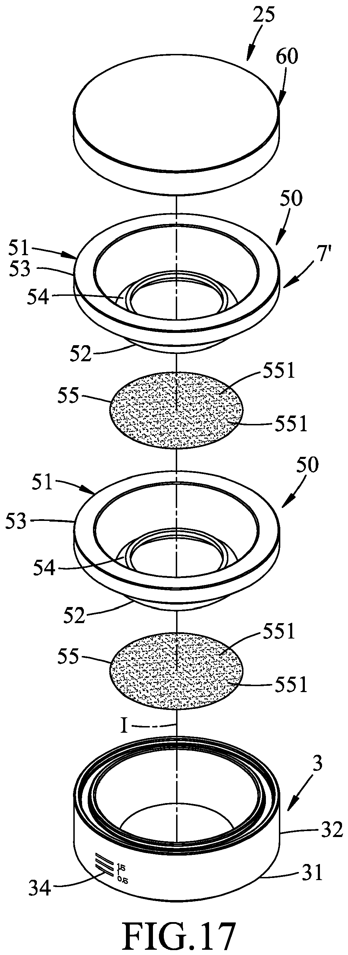

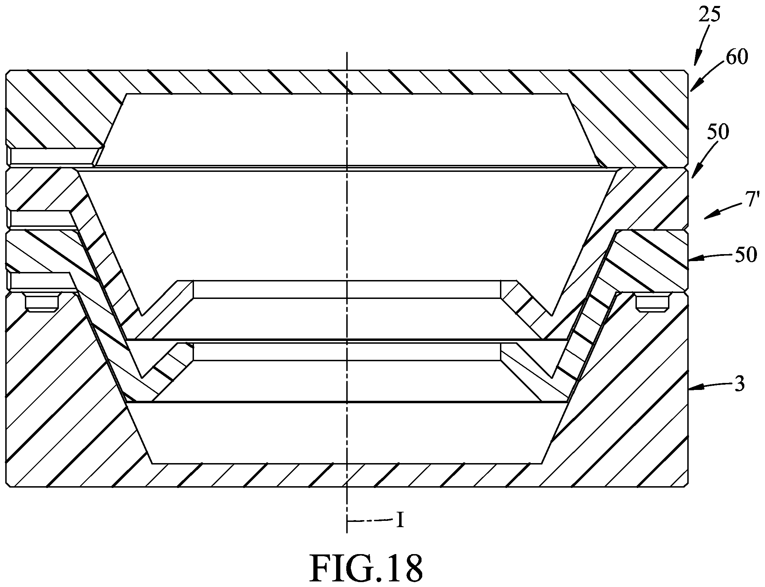

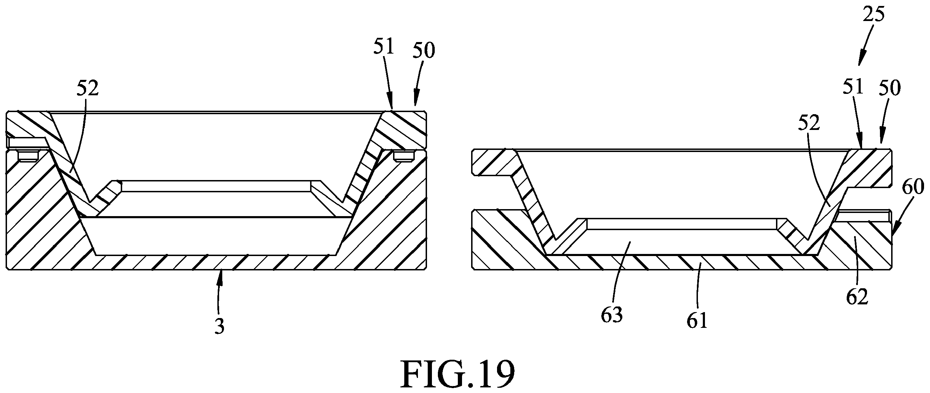

Referring to FIG. 17 to FIG. 19, the sperm sorting device 25 according to the fifth embodiment of the present disclosure includes the base 3, a sorting unit 7', and the cover 60. The sorting unit 7' includes two second sorters 50, one superposed on the other along the axis I.

The cover 60 can be used to cover the upper second sorter 50 (or the lower second sorter 50 if the upper second sorter 50 is not used) or be turned upside down so that the second sleeve 52 of either second sorter 50 can be placed in the inverted top receiving space 63, thereby converting the cover 60 into a supporting platform.

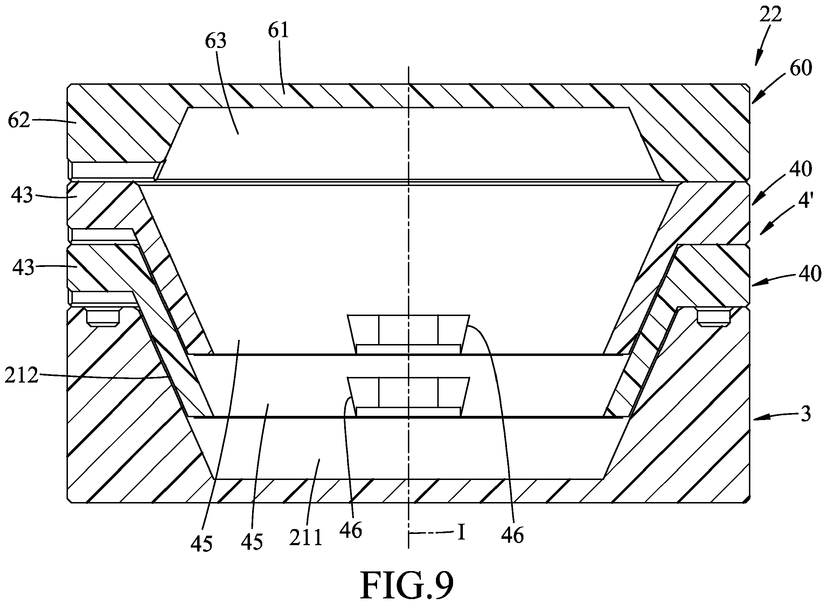

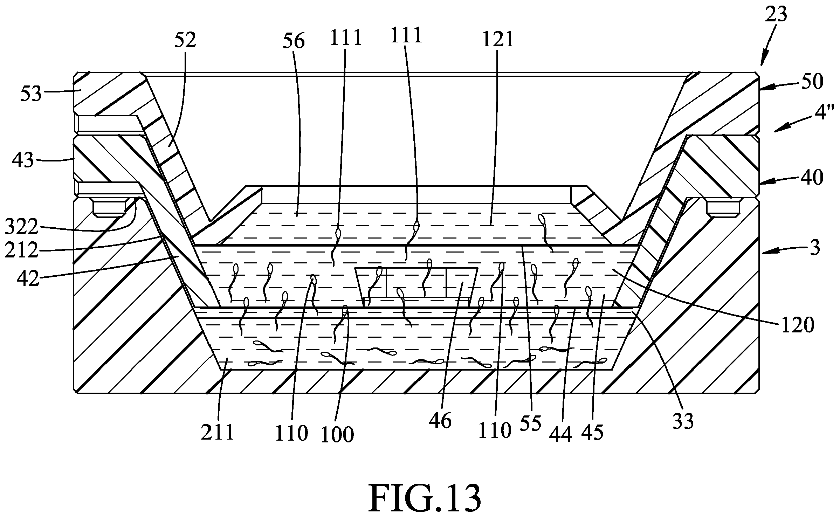

The sperm sorting device 25 has the same function as the sperm sorting device 22 (see FIG. 9) and the sperm sorting device 23 (see FIG. 13).

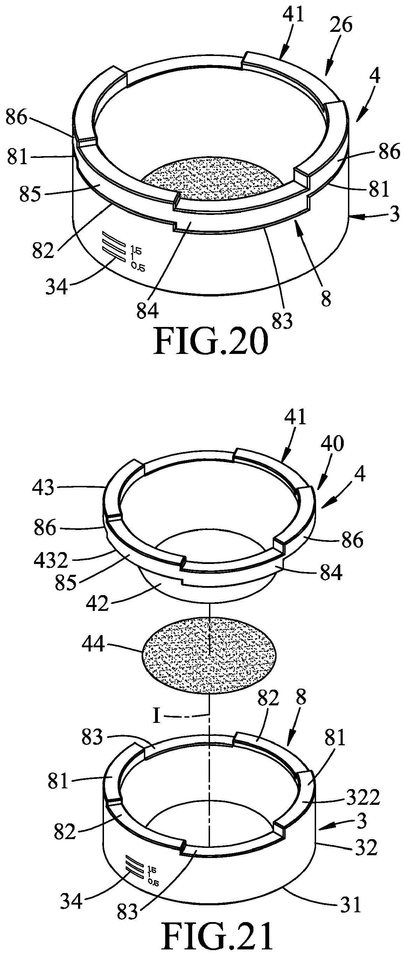

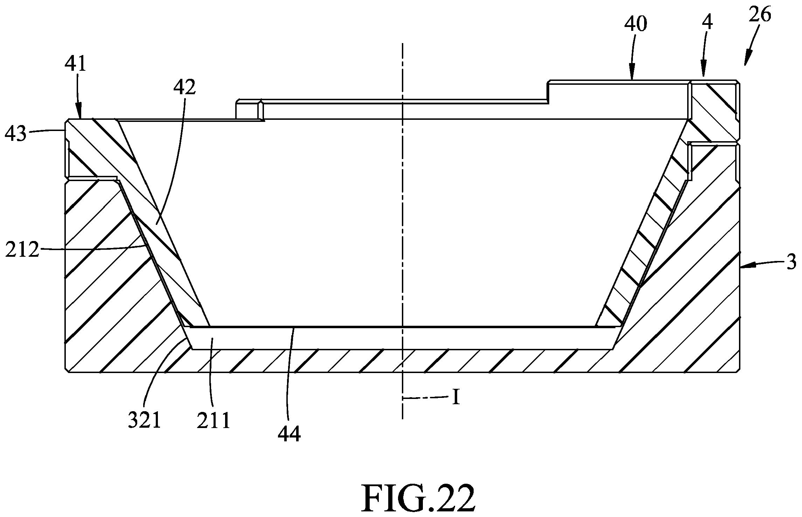

Referring to FIG. 20 to FIG. 22, the sperm sorting device 26 according to the sixth embodiment of the present disclosure includes the base 3, the sorting unit 4, and a height adjustment unit 8.

The height adjustment unit 8 is provided between the top surface 322 of the surrounding wall 32 of the base 3 and the annular bottom surface 432 of the first protruding ring 43 of the first mounting member 41 and includes two first step portions 81, two second step portions 82, two third step portions 83, two first sitting portions 84, two second sitting portions 85, and two third sitting portions 86. The two first step portions 81 are provided on the top surface 322 and are diametrically opposite to each other. The two second step portions 82 are provided on the top surface 322, are connected to the two first step portions 81 respectively, have a lower height along the axis I than the two first step portions 81, and are diametrically opposite to each other. The two third step portions 83 are provided on the top surface 322, are each connected between one of the two first step portions 81 and one of the two second step portions 82, have a lower height along the axis I than the two second step portions 82, and are diametrically opposite to each other. The two first sitting portions 84, the two second sitting portions 85, and the two third sitting portions 86 are provided on the annular bottom surface 432 and correspond to the first step portions 81, the second step portions 82, and the third step portions 83. The two first sitting portions 84 are diametrically opposite to each other. The two second sitting portions 85 are connected to the two first sitting portions 84 respectively and are diametrically opposite to each other. The two third sitting portions 86 are each connected between one of the two first sitting portions 84 and one of the two second sitting portions 85 and are diametrically opposite to each other. Each first step portion 81, each second step portion 82, and each third step portion 83 have a central angle of 60°. Each first sitting portion 84, each second sitting portion 85, and each third sitting portion 86 also have a central angle of 60°. Both the top surface 322 and the annular bottom surface 432 are step-like surfaces.

When the two first sitting portions 84, the two second sitting portions 85, and the two third sitting portions 86 sit on the two third step portions 83, the two second step portions 82, and the two first step portions 81 respectively, the first mounting member 41 is in a state in which it has been rotated through 0° with respect to the base 3. In this state, the sperm sorting device 26 has a first height along the axis I, the first passage gap 212 has a first air capacity, and the first specimen chamber 211 has a first volume.

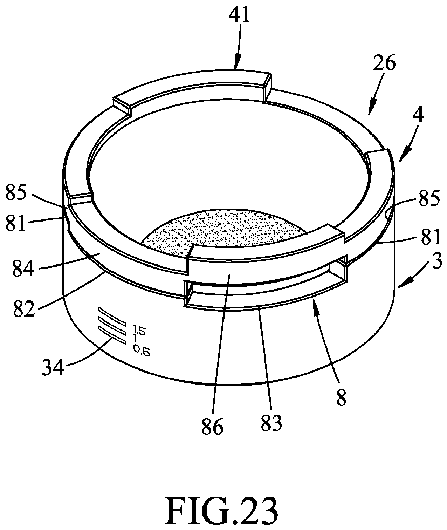

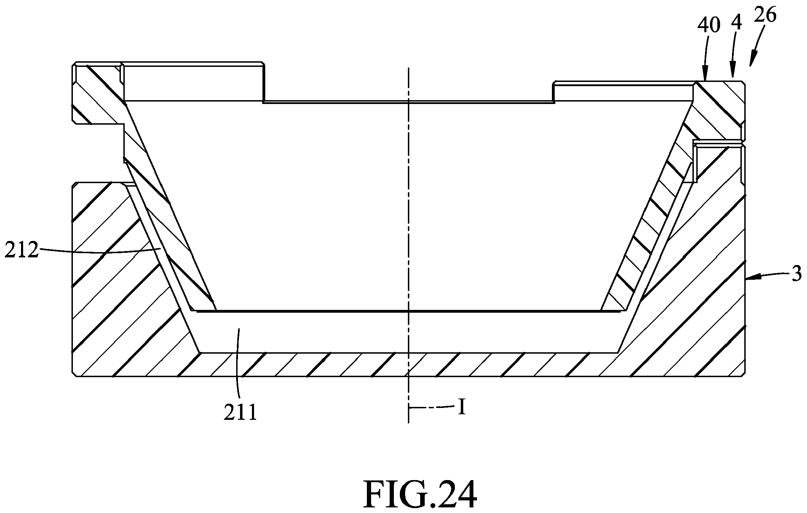

Referring to FIG. 23 and FIG. 24, when the first mounting member 41 has been rotated through 60° with respect to the base 3 such that the two first sitting portions 84, the two second sitting portions 85, and the two third sitting portions 86 sit on the two second step portions 82, the two first step portions 81, and the two third step portions 83 respectively, the sperm sorting device 26 has a second height along the axis I, the first passage gap 212 has enlarged and has a second air capacity, and the first specimen chamber 211 has a second volume. The second height is greater than the first height, the second volume is greater than the first volume, and the second air capacity is greater than the first air capacity.

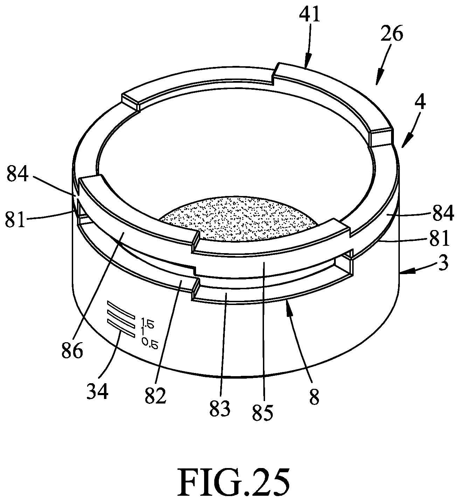

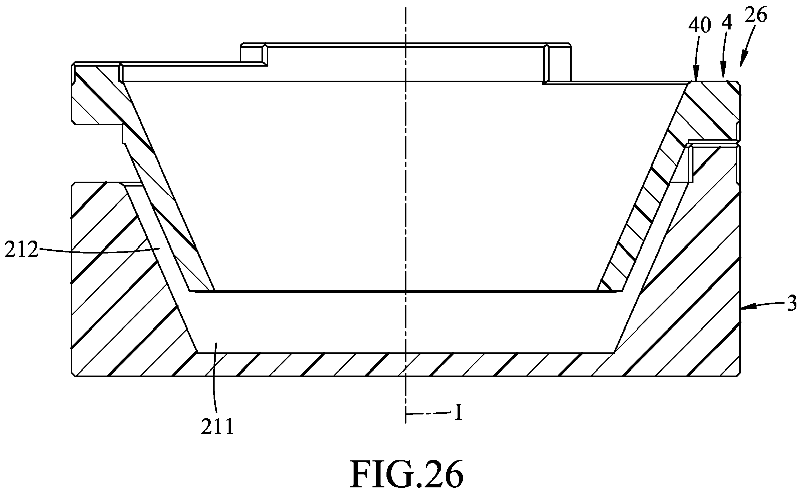

Referring to FIG. 25 and FIG. 26, when the first mounting member 41 has been rotated through 120° with respect to the base 3 such that the two first sitting portions 84, the two second sitting portions 85, and the two third sitting portions 86 sit on the two first step portions 81, the two third step portions 83, and the two second step portions 82 respectively, the sperm sorting device 26 has a third height along the axis I, the first passage gap 212 has enlarged and has a third air capacity, and the first specimen chamber 211 has a third volume. The third height is greater than the second height, the third volume is greater than the second volume, and the third air capacity is greater than the second air capacity.

It is worth noting that the height adjustment unit 8 is not necessarily provided with exactly two first step portions 81, two second step portions 82, two third step portions 83, two first sitting portions 84, two second sitting portions 85, and two third sitting portions 86. There may be a different number of step portions and sitting portions instead. Moreover, it is not required that each step portion/sitting portion have a central angle of 60°, nor is it required that each step portion/sitting portion be diametrically opposite to another step portion/sitting portion.

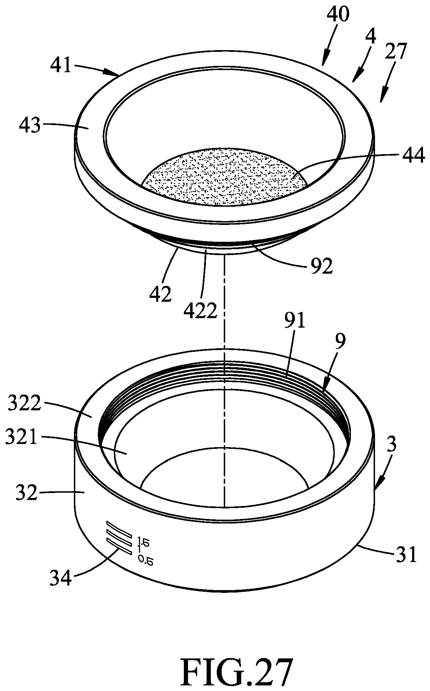

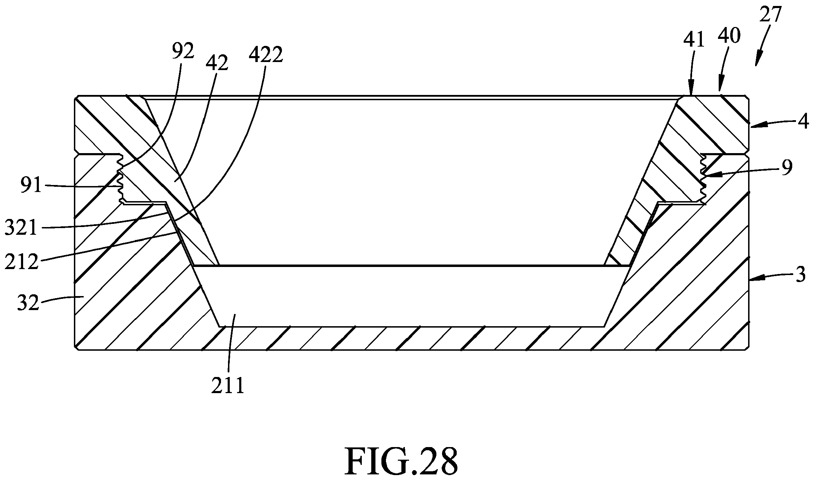

Referring to FIG. 27 and FIG. 28, the sperm sorting device 27 according to the seventh embodiment of the present disclosure includes the base 3, the sorting unit 4, and a height adjustment unit 9.

The height adjustment unit 9 is provided between the inner wall surface 321 of the surrounding wall 32 of the base 3 and the first outer sleeve surface 422 of the first sleeve 42 of the first mounting member 41 and includes a first internal thread section 91 and an external thread section 92. The first internal thread section 91 is located on the inner wall surface 321 and is adjacent to the top surface 322. The external thread section 92 is located on the first outer sleeve surface 422 and is adjacent to the first protruding ring 43.

With the external thread section 92 and the first internal thread section 91 threadedly locked/coupled to each other, the first sorter 40 can be moved closer to or farther away from the base 3 to adjust the size of the first passage gap 212 and the volume of the first specimen chamber 211.

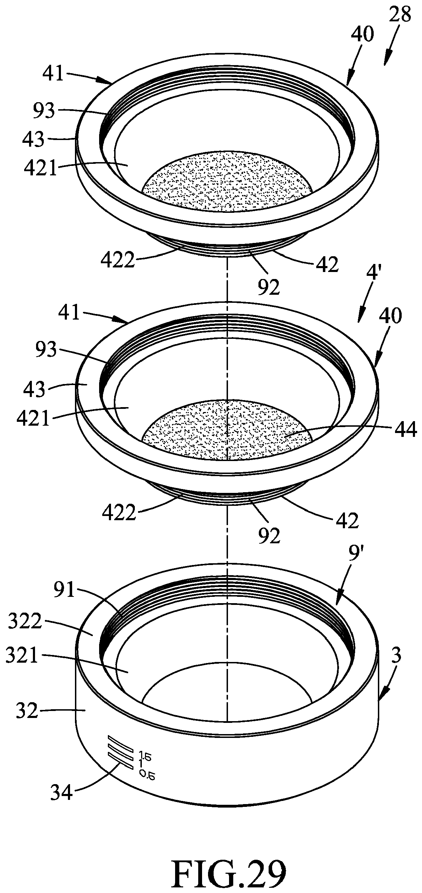

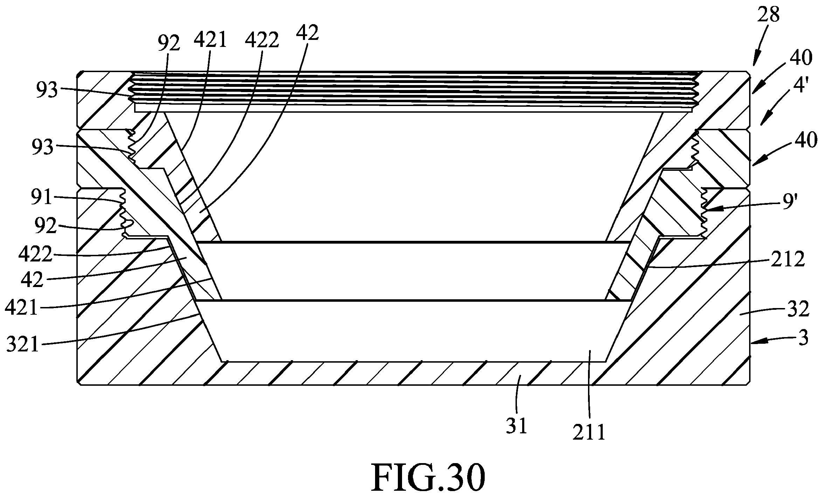

Referring to FIG. 29 and FIG. 30, the sperm sorting device 28 according to the eighth embodiment of the present disclosure includes the base 3, the sorting unit 4', and a height adjustment unit 9'.

The height adjustment unit 9' includes the first internal thread section 91, which is located on the inner wall surface 321 and is adjacent to the top surface 322; the external thread section 92, which is located on the first outer sleeve surface 422, and adjacent to the first protruding ring 43, of each first mounting member 41; and a second internal thread section 93 located on the first inner sleeve surface 421 of the first sleeve 42, and adjacent to the first protruding ring 43, of each first mounting member 41.

With the external thread section 92 of one of the first sorters 40 and the first internal thread section 91 threadedly locked/coupled to each other, the one of the first sorters 40 can be moved closer to, or farther away from, the base 3 to adjust the size of the first passage gap 212 and the volume of the first specimen chamber 211. With the external thread section 92 of the other of the first sorters 40 and the second internal thread section 93 of the one of the first sorters 40 threadedly locked/coupled together, the other of the first sorters 40 can be moved closer to, or farther away from, the one of the first sorters 40 to adjust the volume of the first sorting chamber 45 of the one of the first sorters 40.

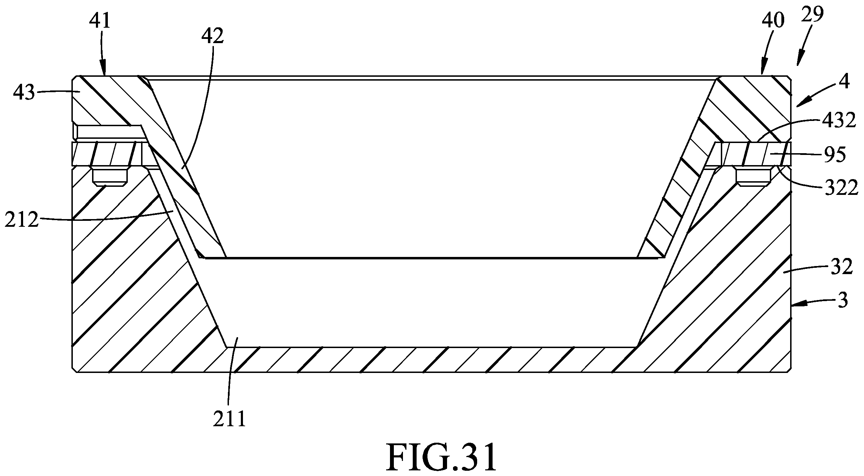

Referring to FIG. 31, the sperm sorting device 29 according to the ninth embodiment of the present disclosure includes the base 3, the sorting unit 4, and a washer 95.

The washer 95 is mounted between the top surface 322 of the base 3 and the annular bottom surface 432 of the first protruding ring 43 of the first sorter 40. The washer 95 is configured to keep the first sorter 40 away from the base 3 so as to increase the size of the first passage gap 212 and the volume of the first specimen chamber 211.

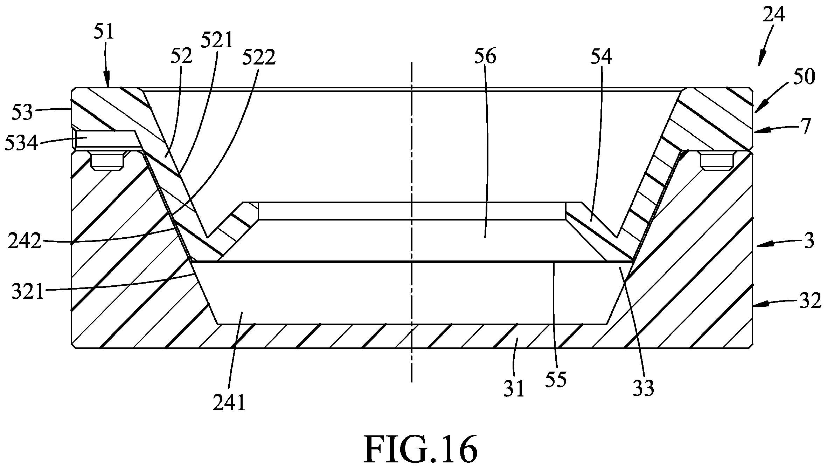

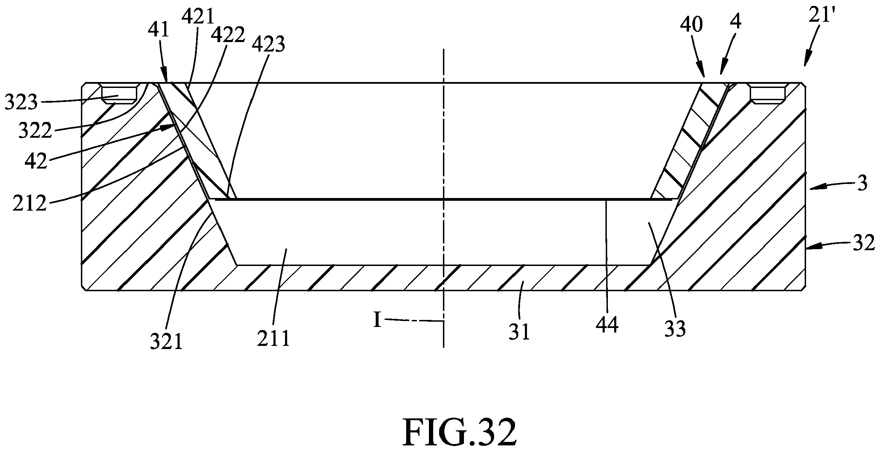

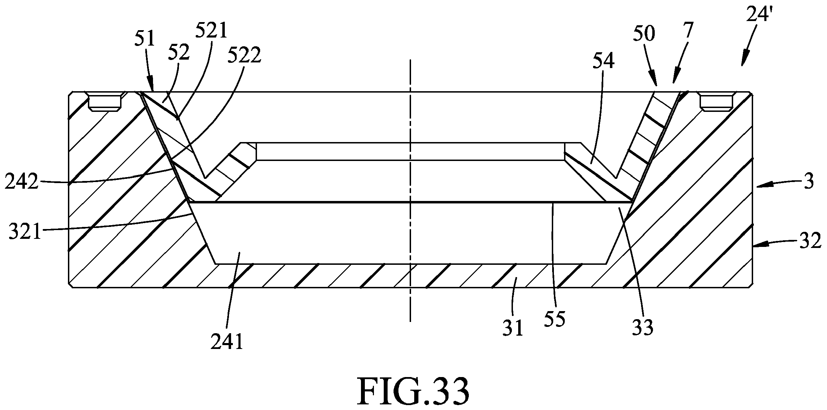

Referring to FIG. 32 in conjunction with FIG. 4, the sperm sorting device 21' according to the tenth embodiment of the present disclosure is similar to the sperm sorting device 21 in that it also includes the base 3 and the sorting unit 4. The sperm sorting device 21' is different from the sperm sorting device 21 in that while the first sorter 40 of the sperm sorting device 21 is positioned by disposing the first protruding ring 43 on the top surface 322 of the surrounding wall 32, the first mounting member 41 of the sperm sorting device 21' dispenses with the first protruding ring 43. The first sorter 40 of the sperm sorting device 21' is positioned by placing the first sleeve 42, which is a truncated cone-shaped sleeve with a wider top than the bottom, into the receiving space 33, which is a truncated-cone shaped space with a wider top than the bottom.Referring to FIG. 33 in conjunction with FIG. 16, the sperm sorting device 24' according to the eleventh embodiment of the present disclosure is similar to the sperm sorting device 24 in that it also includes the base 3 and the sorting unit 7. The sperm sorting device 24' is different from the sperm sorting device 24 in that while the second sorter 50 of the sperm sorting device 24 is positioned by disposing the second protruding ring 53 on the top surface 322 of the surrounding wall 32, the second mounting member 51 of the sperm sorting device 24' dispenses with the second protruding ring 53. The second sorter 50 of the sperm sorting device 24' is positioned by placing the second sleeve 52, which is a truncated cone-shaped (e.g., a partial shell of a frustum-shaped) sleeve with a wider top than the bottom, into the receiving space 33, which is truncated cone-shaped space with a wider top than the bottom.

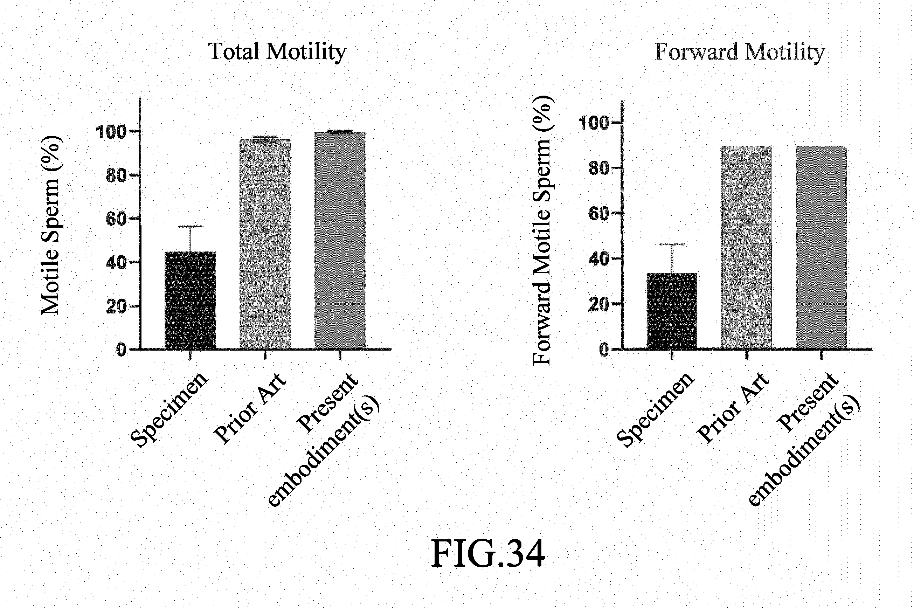

Referring to FIG. 34, the total motility and forward motility of the motile sperms 110 obtained according to the present disclosure, of sperms obtained from the source semen specimen, and of sperms obtained with a prior art device were compared. The experimental results show that the motile sperms 110 obtained according to the disclosure had significantly higher total motility and forward motility. In the statistical charts in FIG. 34, motile sperms (%) = the number of motile sperms/the total number of sperms, and forwardly motile sperms (%) = the number of forwardly motile sperms/the total number of sperms. A t-test was used, in which p < 0.05 was regarded as significant. The symbol ^{∗} indicates p < 0.05, the symbol ^{∗∗∗} indicates p < 0.001, and the symbol ^{∗∗∗∗} indicates p < 0.0001.

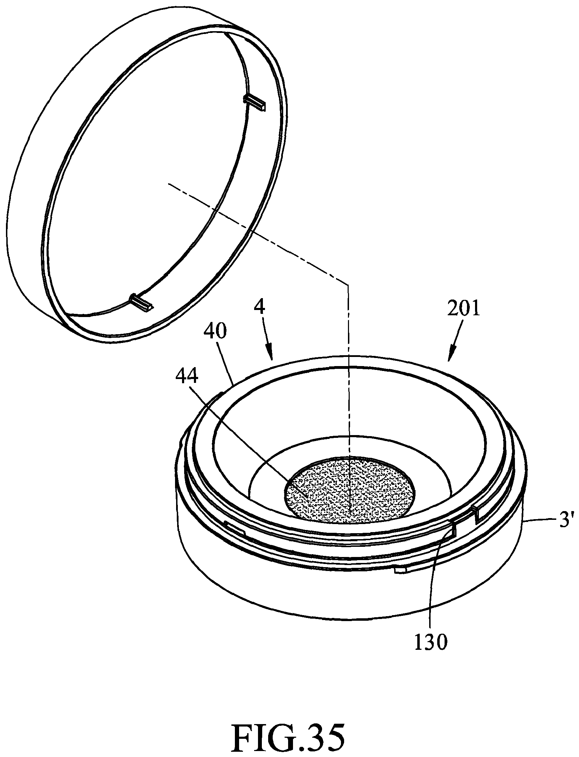

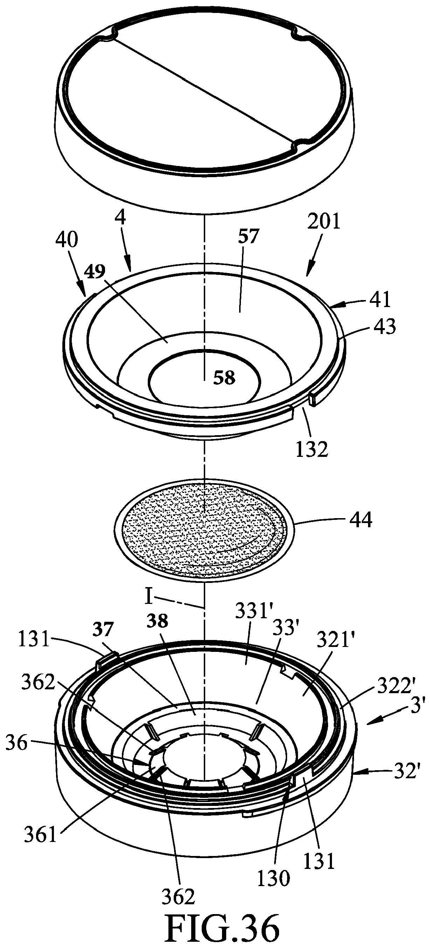

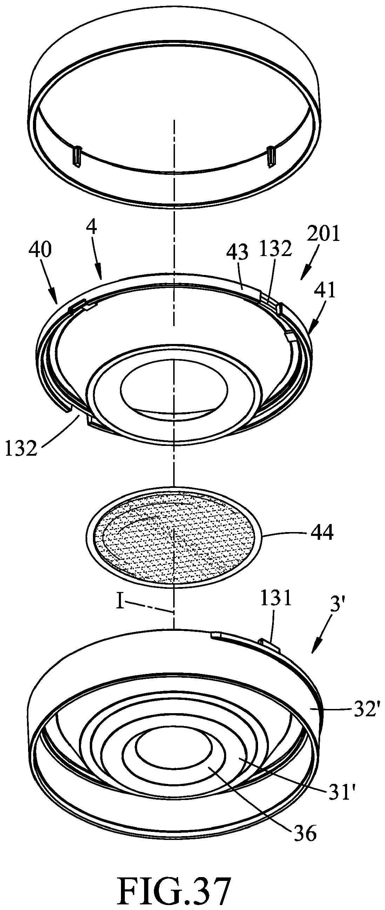

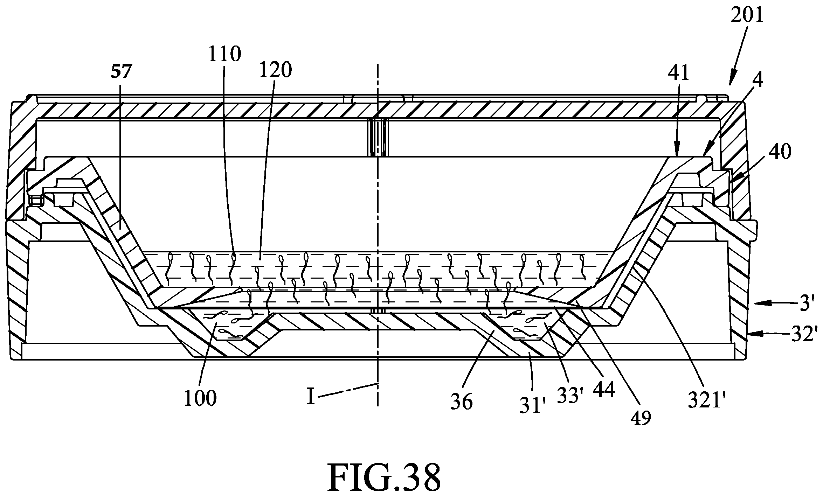

Referring to FIG. 35 to FIG. 38, the sperm sorting device 201 according to the twelfth embodiment of the present disclosure includes a base 3', the sorting unit 4, and an interlocking unit 130.

The base 3' includes a bottom wall 31', a surrounding wall 32' extending from the periphery of the bottom wall 31' toward the top end of the base 3' and surrounding an axis I, an intermediate ring 37, and a central protruding block 36 that extends, and whose diameter is gradually reduced, from a central portion of the bottom wall 31' toward the top end of the base 3' along the axis I.

The central protruding block 36 is a truncated cone-shaped block (e.g., a partial shell of a frustrum/truncated cone) with a wider bottom than the top and has a truncated cone-shaped surface 361 and a plurality of spaced-apart ribs 362 that are provided on the truncated cone-shaped surface 361 at equal intervals (e.g., uniformly spaced apart). In some embodiments, the central protruding block 36 may not include the plurality of spaced-apart ribs 362 or may include another number of ribs 362 than those shown in FIG. 35-38.

The bottom wall 31' surrounds the central protruding block 36 and is coaxial to the surrounding wall 32'. Together, the bottom wall 31' and the central protruding block 36 may be referred to as the bottom of the base 3' The intermediate ring 37 that connects to an external edge of the bottom wall 31' via a first inner wall surface 38. The intermediate ring 37 has a diameter smaller than the diameter of the surrounding wall 32' but larger than the diameter of the bottom wall 31'. The truncated cone-shaped surface 361, the bottom wall 31', and the first inner wall surface 38 together form a specimen space configured to receive a semen specimen.

The bottom wall 31' and the surrounding wall 32' jointly define a receiving space 33' that has an opening 331'. The surrounding wall 32' has a second inner wall surface 321' and a top surface 322'. The surrounding wall 32' is a coaxial cylinder that connects at a top portion to a top edge of the intermediate ring 37 via the second inner wall surface 321'. The first inner wall surface 38 and the second inner wall surface 321'are each angled towards a center of the base 3'. In some embodiments, the first inner wall surface 38 includes a set of uniformly spaced ribs.

As in the first embodiment, the sorting unit 4 in the twelfth embodiment includes the first sorter 40, which has the first mounting member 41 and the first sorting filter 44. The sorting unit 4 is structurally adapted to sit on top of the base 3' and includes a first protruding ring 43 and a sorting ring 49. The first protruding ring 43 is on a periphery of the sorting unit 4 as part of the first mounting member 41 and connects to an outer portion of the sorting ring 49 via an inner sleeve surface. The protruding ring is structurally adapted to sit on top of the base 3' as part of the first mounting member 41. The sorting ring 49 is situated at the bottom of the sorting unit 4 and includes a central hole 58. The first sorting filter 44 spans the central hole 58 and filters the semen specimen. The side of the sorting ring 49 facing the sorting filter 44 is beveled at an angle that increases a permeable surface of the first sorting filter 44.

The first sorting filter 44 includes a permeable portion (e.g., a membrane) and a fixed portion. The permeable portion is a circular section in the center of the first sorting filter 44 and may bulge in the vertical direction (based on axis I). The permeable portion is made of a filtering material with a plurality of micropores that allow motile sperm to swim through. The fixed portion surrounds the permeable portion and is made of less flexible material than the permeable portion. The fixed portion contacts the sorting 49 when the sorting unit 4 is placed on top of the base 3'. The first sorting filter 44 is fixed to a sleeve that includes the inner sleeve surface, such that the first sorting filter 44 and inner sleeve surface jointly define a sorting chamber with an open top end at the top of the first sorter 40. The first sorting filter 44 contacts the specimen space when the sleeve is placed in the coaxial cylinder of the surrounding wall 32' of the base 3'.

The interlocking unit 130 is provided between the first protruding ring 43 of the first mounting member 41 and the top surface 322'of the surrounding wall 32' and includes two engaging blocks 131 and two engaging grooves 132. The engaging blocks 131 are provided on the top surface 322' (e.g., at a top of the surrounding wall 32'). The engaging grooves 132 are provided in the first protruding ring 43 and correspond to the engaging blocks 131 respectively. The interlocking unit 130 can prevent the first sorter 40 and the base 3' from rotating with respect to each other so that the first sorter 40 can be positioned more securely and operated with greater ease. A bottom portion of the sorting ring 49 may be vertically displaced by the first sorting filter 44 from a top of the intermediate ring 37 when the engaging grooves 132 in contact with the engaging blocks 131. It is worth noting that the engaging grooves 132 may be provided in the top surface 322' instead while the engaging blocks 131 are provided on the first protruding ring 43. This alternative configuration works just as well as that of the interlocking unit 130 illustrated herein.

The sperm sorting device 201 is so designed that, thanks to the central protruding block 36 on the bottom wall 31' of the base 3' and the spaced-apart ribs 362, the semen specimen 100 can be applied, in drops, onto the truncated cone-shaped surface 361 between each two adjacent spaced-apart ribs 362 so as to coat the entire truncated cone-shaped surface 361 evenly, which helps increase the uniformity of the semen specimen 100 in the receiving space 33'. Once the drop-by-drop application of the semen specimen 100 is completed, and the first sorter 40 is positioned on the base 3', the truncated cone-shaped surface 361 allows air bubbles on the surface of the semen specimen 100 to gather at the top end of the central protruding block 36 and be let out easily through the first sorting filter 44. Moreover, while the first sorting medium 120 and the motile sperms 110 are being drawn by suction, the spaced-apart ribs 362 on the central protruding block 36 will hinder the flow of the semen specimen 100, thereby preventing the impurities in the semen specimen 100 from being drawn out through the first sorting filter 44. The impurities may also be stopped at the junctions between the truncated cone-shaped surface 361 and the spaced-apart ribs 362 and therefore will not be drawn out through the first sorting filter 44. Consequently, sorting quality is enhanced.

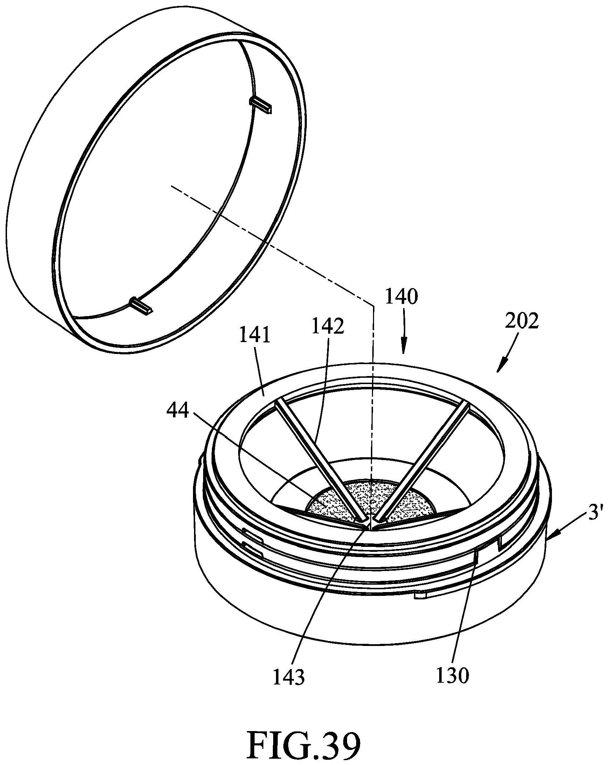

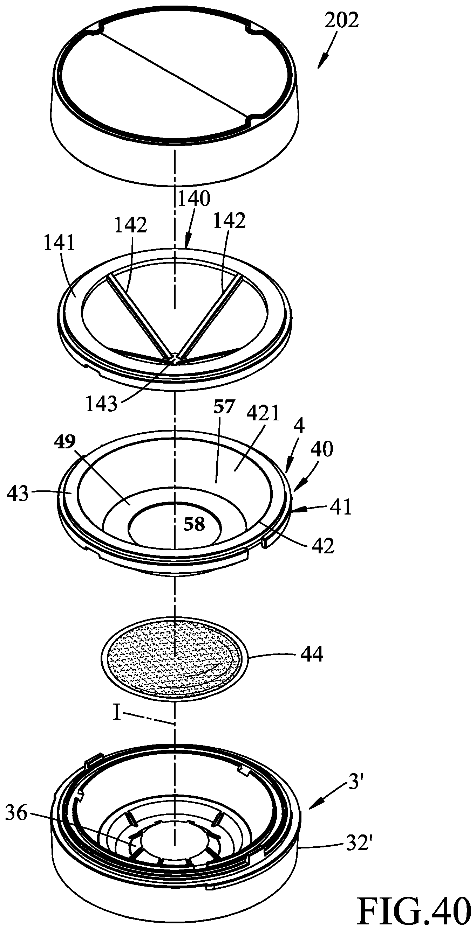

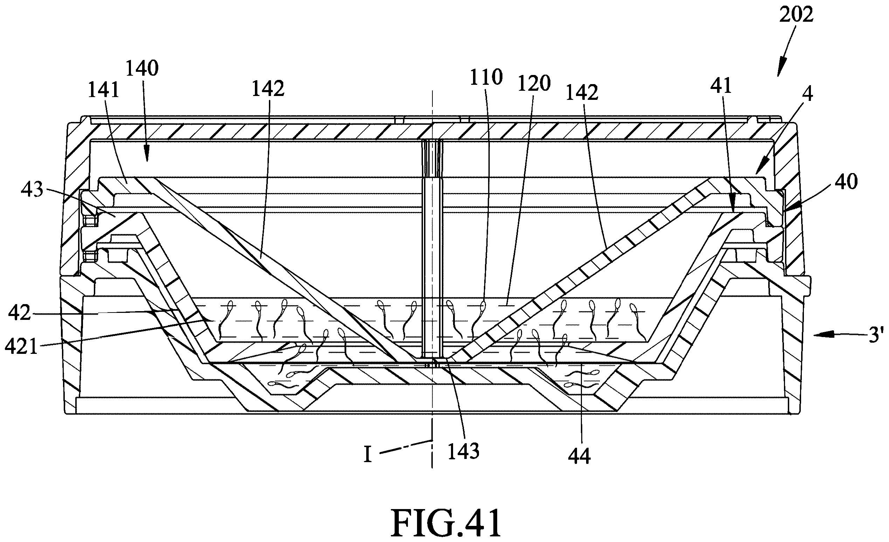

Referring to FIG. 39 to FIG. 41, the sperm sorting device 202 according to the thirteenth embodiment of the present disclosure includes the base 3', the sorting unit 4, the interlocking unit 130, and an auxiliary frame 140 for a liquid taking device.

The sperm sorting device 202 is different from the sperm sorting device 201 (see FIG. 35 to FIG. 38) in that the sperm sorting device 202 is additionally provided with the auxiliary frame 140 for a liquid taking device. The auxiliary frame 140 for a liquid taking device includes a top placement ring 141, a plurality of connecting ribs 142, and a bottom liquid-taking block 143. The top placement ring 141 is configured to be placed on the first protruding ring 43 of the first mounting member 41. The connecting ribs 142 extend from the top placement ring 141 toward the bottom end of the auxiliary frame 140 for a liquid taking device, converge toward the axis I, and are located on the first inner sleeve surface 421 of the first sleeve 42. The bottom liquid-taking block 143 is connected to the bottom ends of the connecting ribs 142 and is located at the top end of the first sorting filter 44.

It is worth noting that the auxiliary frame 140 for a liquid taking device and the first mounting member 41 may be two separate components as described above or form a single structure. In the latter case, the auxiliary frame 140 for a liquid taking device only has to include the connecting ribs 142 and the bottom liquid-taking block 143, with the connecting ribs 142 provided in the first inner sleeve surface 421 and converging toward the axis I, and the bottom liquid-taking block 143 connected to the bottom ends of the connecting ribs 142 and located at the top end of the first sorting filter 44.

To use the sperm sorting device 202, the needle of a liquid taking device (e.g., a syringe, not shown) is pressed against the bottom liquid-taking block 143 so that the first sorting medium 120 and the motile sperms 110 can be drawn with the liquid taking device while the first sorting filter 44 is protected from being punctured by accident during the process.

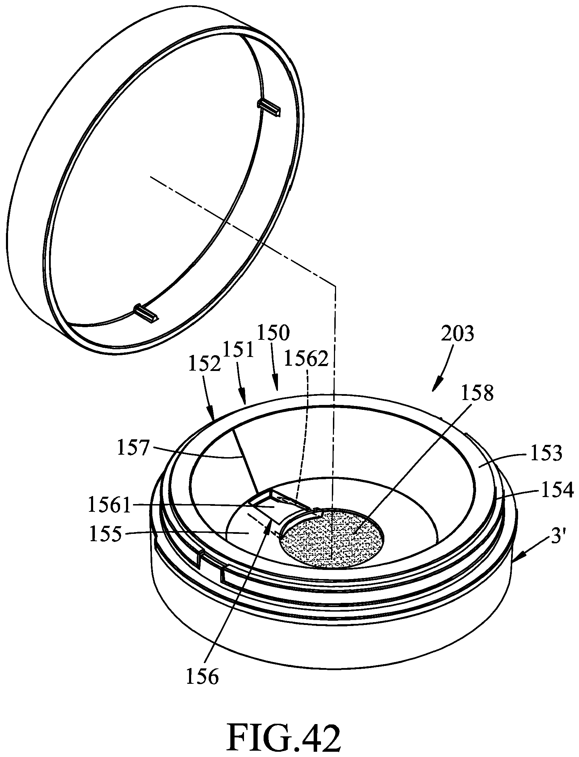

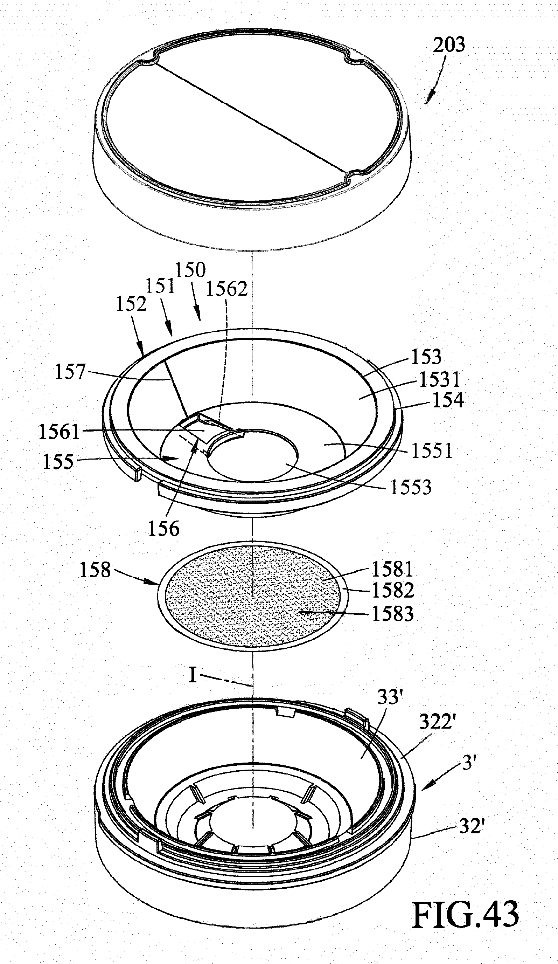

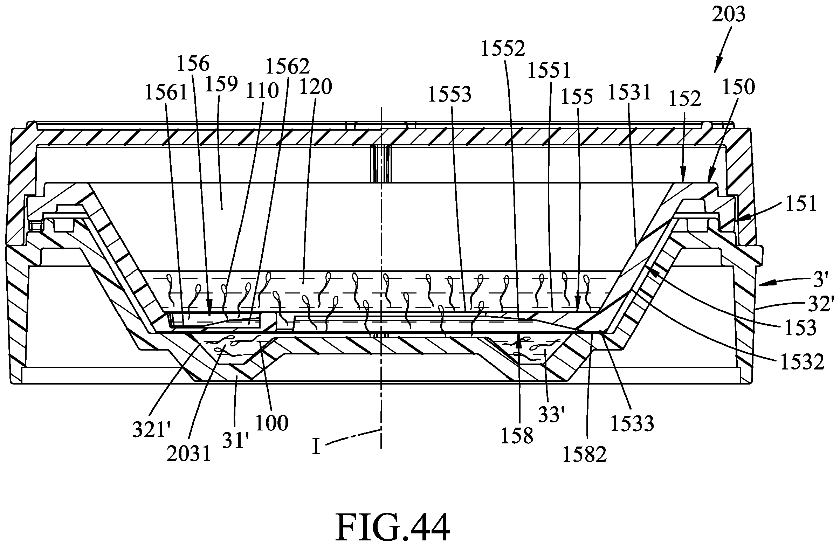

Referring to FIG. 42 to FIG. 44, the sperm sorting device 203 according to the fourteenth embodiment of the present disclosure includes the base 3' and a sorting unit 150.

The sorting unit 150 includes a third sorter 151, and the third sorter 151 has a third mounting member 152 and a third sorting filter 158. The sorting unit 150 is structurally adapted to sit on top of the base 3'.

The third mounting member 152 has a third sleeve 153, a third protruding ring 154, a sorting ring 155, a position-limiting groove 156 for a liquid taking device, and an indicating line 157. An inner portion of the third protruding ring 43 connects to an outer portion of the sorting ring 155 via a third inner sleeve surface 1531. The third sleeve 153 surrounds the axis I and is configured to be placed in the receiving space 33'. The third protruding ring 154 extends from the top end of the third sleeve 153 in a direction away from the axis I and is configured to be positioned on the top surface 322' of the surrounding wall 32'.

The sorting ring 155 extends from the bottom end of the third sleeve 153 toward the axis I and includes a central hole 1553 that allows motile sperm from the semen specimen in the base 3' to move vertically through. The side of the sorting ring 155 facing the sorting filter 158 is beveled at an angle to increase a permeable surface of the third sorting filter 158, which, when placed under the sorting unit 150, spans the central hole 1553. The position-limiting groove 156 for a liquid taking device is provided at the sorting ring 155 and has an open top end. The indicating line 157 is provided on a third inner sleeve surface 1531 of the third sleeve 153 and corresponds to the position-limiting groove 156 for a liquid taking device.

The third sleeve 153 has the third inner sleeve surface 1531, a third outer sleeve surface 1532 surrounding the third inner sleeve surface 1531, and a third bottom sleeve surface 1533 connecting the third inner sleeve surface 1531 and the third outer sleeve surface 1532.

The sorting ring 155 has an annular top surface 1551, an annular bottom surface 1552, and a central hole 1553 that brings the annular top surface 1551 and the annular bottom surface 1552 into communication with each other. The annular bottom surface 1552 gradually approaches the annular top surface 1551 while extending from the third bottom sleeve surface 1533 of the third sleeve 153 toward the axis I; as a result, the height of the sorting ring 155 is gradually reduced as the sorting ring 155 extends from the bottom end of the third sleeve 153 toward the axis I.

The position-limiting groove 156 for a liquid taking device extends inward from the annular top surface 1551 toward the annular bottom surface 1552 and has a groove bottom portion 1561 and a communicating portion 1562 that brings the groove bottom portion 1561 and the third sorting filter 158 into communication with each other. The width of the position-limiting groove 156 for a liquid taking device ranges from 4 µm to 5 mm.

The third sorting filter 158 is fixed on the third bottom sleeve surface 1533 of the third sleeve 153. The third sorting filter 158, the sorting ring 155, and the third inner sleeve surface 1531 jointly define a third sorting chamber 159 that has an open top end. The third sorting filter 158 has a plurality of third micropores 1581 through which the motile sperms 110 are allowed to pass, a fixed portion 1582 surrounding the axis I and fixed on the third bottom sleeve surface 1533 of the third sleeve 153, and a permeable portion 1583 extending from the fixed portion 1582 toward the axis I, wherein the permeable portion 1583 has a level configuration. The permeable portion 1583 spans the central hole 1553 and contacts the third bottom sleeve surface 1533.

When the third sleeve 153 of the third mounting member 152 is placed in the receiving space 33', a third specimen chamber 2031 for receiving the semen specimen 100 is jointly defined by the third sorting filter 158, the bottom wall 31', and the second inner wall surface 321' of the surrounding wall 32'.

Thanks to the sperm sorting device 203 having the sorting ring 155 and the position-limiting groove 156 for a liquid taking device, an operator only has to insert the needle of a liquid taking device (e.g., a syringe, not shown) into the position-limiting groove 156 for a liquid taking device and press the needle against the groove bottom portion 1561, in order to draw the first sorting medium 120 and the motile sperms 110 with high motility by suction while preventing the needle of the liquid taking device from puncturing the third sorting filter 158. Moreover, as the position-limiting groove 156 for a liquid taking device is in communication with the third sorting filter 158 through the communicating portion 1562, the first sorting medium 120 that is located between the third sorting filter 158 and the annular bottom surface 1552 of the sorting ring 155 can be drawn out, thereby reducing residues of the first sorting medium 120. The indicating line 157 can guide an operator in inserting the needle of a liquid taking device into the position-limiting groove 156 for a liquid taking device.

In addition, as the annular bottom surface 1552 gradually approaches the annular top surface 1551 while extending from the third bottom sleeve surface 1533 of the third sleeve 153 toward the axis I, the area over which the fixed portion 1582 of the third sorting filter 158 is fixed to the third bottom sleeve surface 1533 can be reduced to increase the permeable area of the third sorting filter 158.

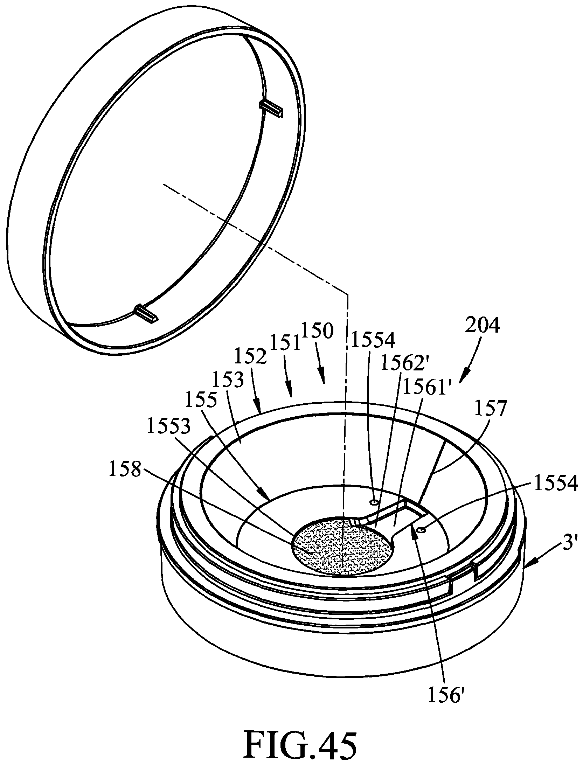

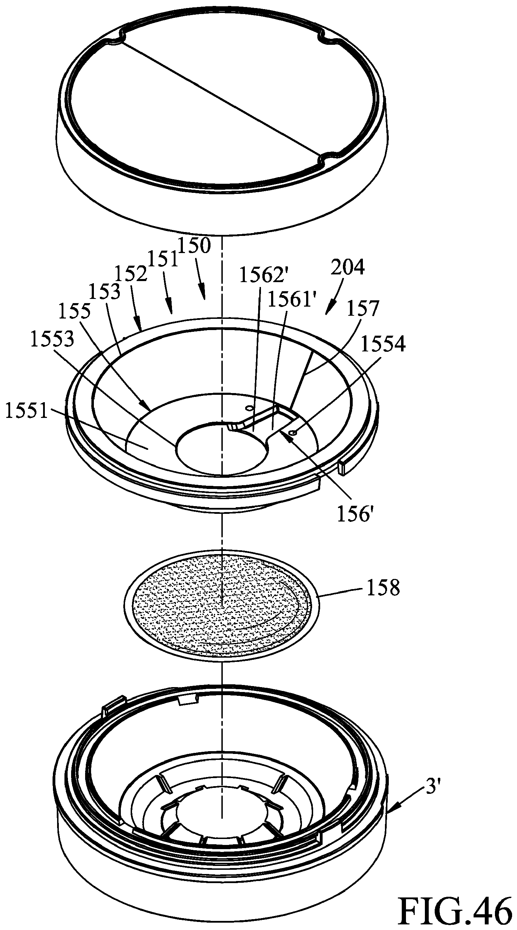

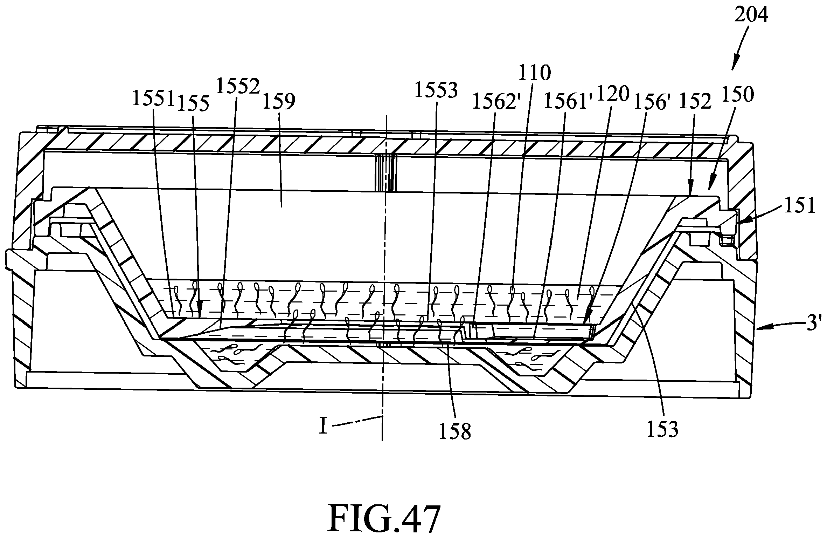

Referring to FIG. 45 to FIG. 47, the sperm sorting device 204 according to the fifteenth embodiment of the present disclosure includes the base 3' and the sorting unit 150.

The sperm sorting device 204 is different from the sperm sorting device 203 (see FIG. 42 to FIG. 44) in that the position-limiting groove 156' for a liquid taking device of the third mounting member 152 of the sperm sorting device 204 not only extends from the annular top surface 1551 toward the annular bottom surface 1552, but also has a groove bottom portion 1561' and a communicating portion 1562' that brings the groove bottom portion 1561' and the central hole 1553, and hence the position-limiting groove 156' for a liquid taking device and the third sorting filter 158, into communication with each other. Thus, the inside space of the position-limiting groove 156' opens to the central hole 1553 and the groove bottom portion 1561' contacts the third sorting filter 158. This configuration is equally capable of preventing the needle of a liquid taking device (not shown) from puncturing the third sorting filter 158, and of reducing residues of the first sorting medium 120 by allowing the first sorting medium 120 that is located between the third sorting filter 158 and the annular bottom surface 1552 of the sorting ring 155 to be drawn out.

Furthermore, the sorting ring 155 has a plurality of vent holes 1554 that bring the annular top surface 1551 and the annular bottom surface 1552 into communication with each other. The vent holes 1554 are separated by the position-limiting groove 156', which is indented below the annular top surface 1551. Thus, when an operator applies the first sorting medium 120, in drops, onto the third sorting filter 158, air bubbles generated between the annular bottom surface 1552 and the third sorting filter 158 can be let out through the vent holes 1554 to ensure that the entire third sorting filter 158 is coated with the first sorting medium 120, thereby allowing the motile sperms 110 that are swimming upward to pass through the third sorting filter 158 with success.

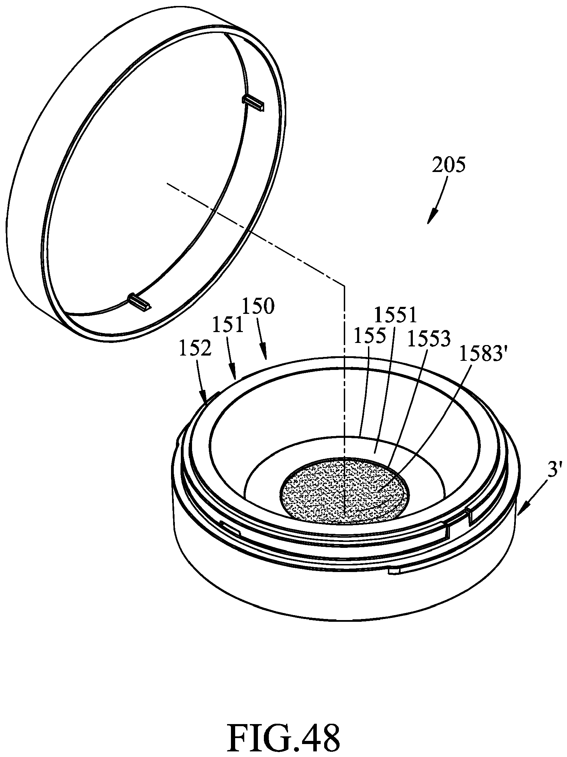

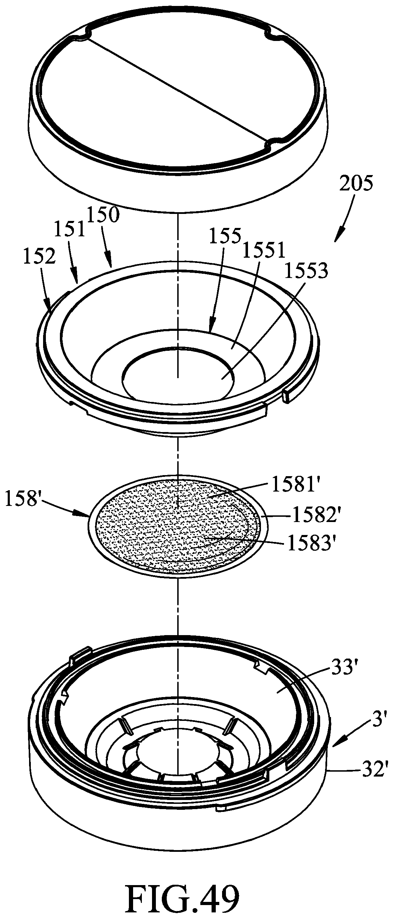

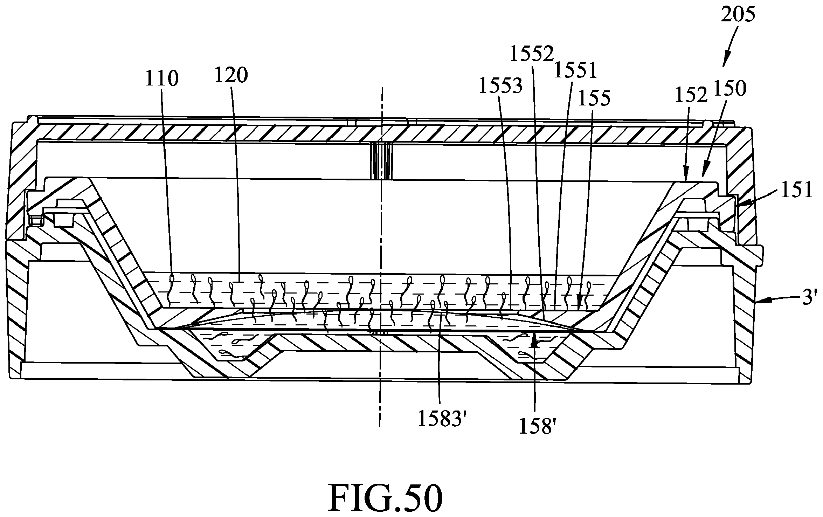

Referring to FIG. 48 to FIG. 50, the sperm sorting device 205 according to the sixteenth embodiment of the present disclosure includes the base 3' and the sorting unit 150.

The sperm sorting device 205 is different from the sperm sorting device 203 (see FIG. 42 to FIG. 44) in that the sorting ring 155 of the third mounting member 152 of the sperm sorting device 205 is not provided with the position-limiting groove 156 for a liquid taking device, and that the third sorting filter 158' of the sperm sorting device 205 has a plurality of third micropores 1581', a fixed portion 1582', and a permeable portion 1583' that extends from the fixed portion 1582' toward the axis I and bulges toward the top end of the third mounting member 152. The permeable portion 1583 spans the central hole 1553 and contacts the third bottom sleeve surface 1533.

To use the sperm sorting device 205, an operator only has to press the needle of a liquid taking device (e.g., a syringe, not shown) against the annular top surface 1551 of the sorting ring 155, and the first sorting medium 120 and the motile sperms 110 with high motility can be drawn, with the third sorting filter 158 protected from being punctured by the needle of the liquid taking device.

As the permeable portion 1583' of the sperm sorting device 205 bulges toward the top end of the third mounting member 152, the permeable area is increased, and the space between the permeable portion 1583' and the annular bottom surface 1552 of the sorting ring 155 is made smaller to reduce residues of the first sorting medium 120 that may lie beneath the annular bottom surface 1552 of the sorting ring 155.

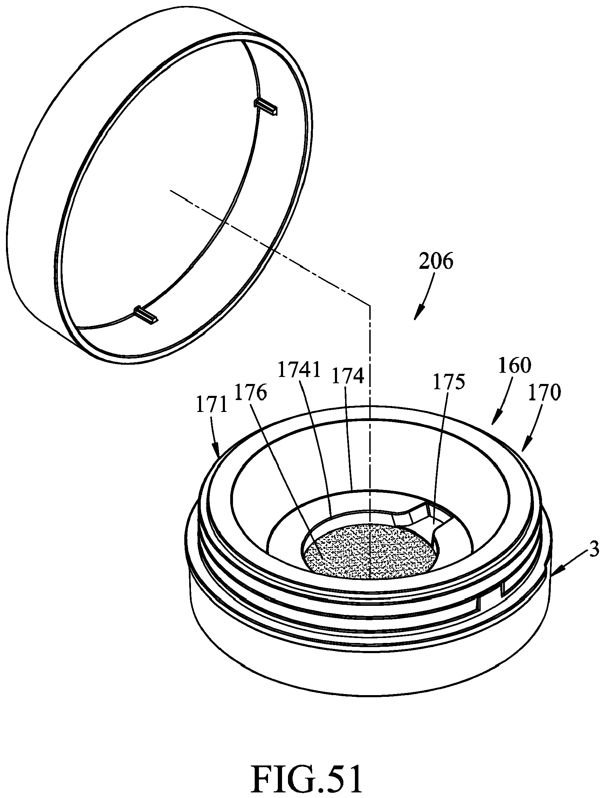

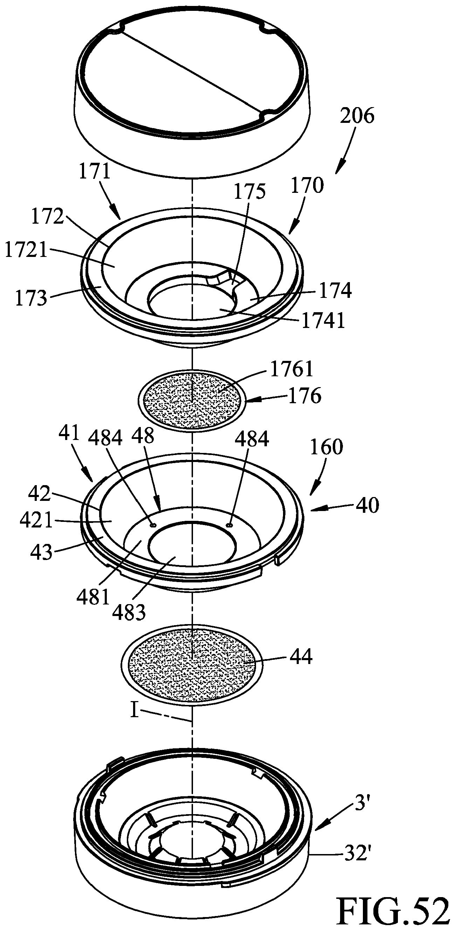

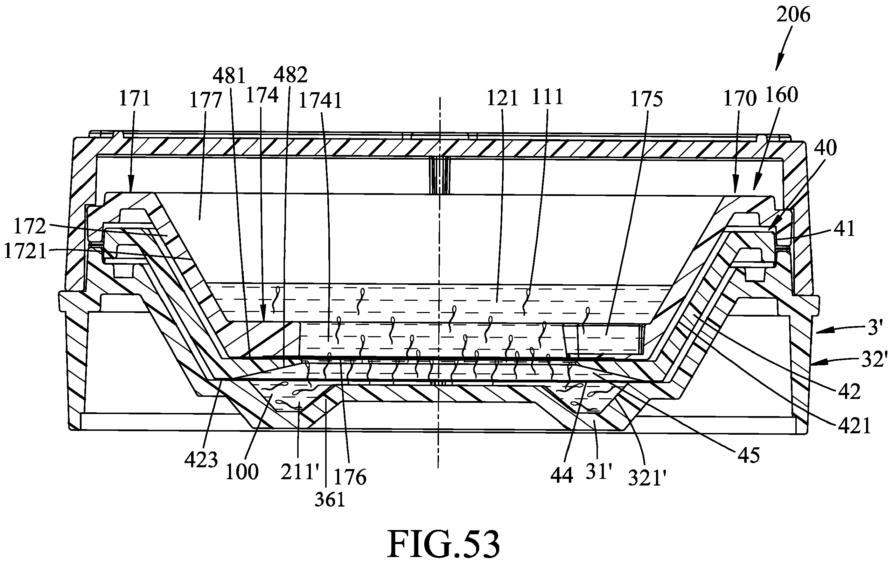

Referring to FIG. 51 to FIG. 53, the sperm sorting device 206 according to the seventeenth embodiment of the present disclosure includes the base 3' and a sorting unit 160.

The sorting unit 160 includes the first sorter 40 and a fourth sorter 170. As in the first embodiment, the first sorter 40 has the first mounting member 41 and the first sorting filter 44. The first sorting filter 44, the bottom wall 31', and the second inner wall surface 321' of the surrounding wall 32' jointly define the first specimen chamber 211' (e.g., the specimen space once enclosed by the first sorting filter 44) for receiving the semen specimen 100, and the first sorting filter 44 and the first inner sleeve surface 421 of the first sleeve 42 jointly define the first sorting chamber 45 with an open top end. An inner portion of the first protruding ring 43 connects to an outer portion of the sorting ring 155 via the first inner sleeve surface 421.

The first mounting member 41 includes the sorting ring 48 that extends from the bottom end of the first sleeve 42 toward the axis I. The sorting ring 48 has an annular top surface 481, an annular bottom surface 482, a central hole 483 that brings the annular top surface 481 and the annular bottom surface 482 into communication with each other, and a plurality of vent holes 484 that also bring the annular top surface 481 and the annular bottom surface 482 into communication with each other. The first sorting filter 44 spans the central hole 483 and filters the semen specimen. The side of the sorting ring 155 facing the sorting filter 44 is beveled at an angle that increase the permeable surface of the first sorting filter 44. For instance, the annular bottom surface 482 gradually approaches the annular top surface 481 while extending from the first bottom sleeve surface 423 of the first sleeve 42 toward the axis I; as a result, the height of the sorting ring 48 is gradually reduced as the sorting ring 48 extends from the bottom end of the first sleeve 42 toward the axis I.

The fourth sorter 170 has a fourth mounting member 171 and a fourth sorting filter 176. The fourth mounting member 171 is structurally adapted to sit on top of the first mounting member 41.

The fourth mounting member 171 has a fourth sleeve 172, a fourth protruding ring 173, a liquid drawing ring 174, and a position-limiting groove 175 for a liquid taking device. The fourth sleeve 172 surrounds the axis I and is configured to be placed in the first sleeve 42. The fourth sleeve 172 connects an inside portion of the fourth protruding ring 173 to an outside portion of the sorting ring 174. The fourth protruding ring 173 extends from the top end of the fourth sleeve 172 in a direction away from the axis I and is configured to be disposed on the first protruding ring 43. For example, in some embodiments, an exterior portion of the fourth protruding ring 173 connects to a second set of engaging grooves on the first protruding ring 43 of the first mounting member 41 to prevent the fourth mounting member 171 from moving horizontally with respect to the first mounting member 41. The liquid drawing ring 174 extends from the bottom end of the fourth sleeve 172 toward the axis I and forms a central hole 1741. The position-limiting groove 175 for a liquid taking device is provided at the liquid drawing ring 174, has an open top end, and is in communication with the central hole 1741 such that the inside space of the position-limiting groove 156' opens to the central hole 1553.

The fourth sorting filter 176 is fixed on a fourth bottom sleeve surface of the fourth sleeve 172. The fourth sorting filter 176, the liquid drawing ring 174, and a fourth inner sleeve surface 1721 of the fourth sleeve 172 jointly define a fourth sorting chamber 177 that has an open top end. The fourth sorting filter 176 spans the central hole 1741 and is in communication with the position-limiting groove 175 for a liquid taking device. The fourth sorting filter 176 has a plurality of fourth micropores 1761 through which the motile sperms 111, which have higher motility than motile sperms able to pass through only one sorting filter, are allowed to pass.

The sperm sorting device 206 is configured for carrying out a two-tier sperm sorting operation and is therefore used in a similar way to the sperm sorting device 23 (see FIG. 11 to FIG. 13), the difference being that an operator can insert the needle of a liquid taking device (e.g., a syringe) into the position-limiting groove 175 and draw the second sorting medium 121 and the motile sperms 111, which have higher motility than motile sperms able to pass through only one sorting filter, directly from the position-limiting groove 175 while preventing the needle of the liquid taking device from puncturing the fourth sorting filter 176.

Besides, the first mounting member 41 is provided with the sorting ring 48 and the vent holes 484 so that when an operator applies the first sorting medium 120, in drops, onto the first sorting filter 44, air bubbles generated between the annular bottom surface 482 and the first sorting filter 44 can be let out through the vent holes 484. This ensures that the entire first sorting filter 44 is coated with the first sorting medium 120, thereby allowing the motile sperms 110 that are swimming upward to pass through the first sorting filter 44 with success.

When the liquid drawing ring 174 of the fourth mounting member 171 is so designed that its annular bottom surface gradually approaches its annular top surface while extending from the fourth bottom sleeve surface of the fourth sleeve 172 toward the axis I, the liquid drawing ring 174 may also be provided with a plurality of vent holes that bring the annular top surface and the annular bottom surface of the liquid drawing ring 174 into communication with each other. These vent holes can produce the same effect as the vent holes 484 and may be similarly spaced.

The following are worth noting: (1) In the present disclosure, the first micropores 441 of the first sorting filter 44, the second micropores 551 of the second sorting filter 55, the third micropores 1581 of the third sorting filter 158, and the fourth micropores 1761 of the fourth sorting filter 176 may be ordinary pores or mesh openings formed by weaving. Furthermore, each of the first sorting filter 44, the second sorting filter 55, the third sorting filter 158, and the fourth sorting filter 176 may be a structure with multiple membranes superposed on one another, in some embodiments. (2) The embodiments desscribed not only can be used to sort human sperms, but also is suitable for sorting the sperms of various animals, and the heights of the first specimen chambers 211 and 211', of the second specimen chamber 241, and of the third specimen chamber 2031 can be changed accordingly. For example: 1) to sort the sperms of rams, the heights of the specimen chambers should be not less than 67.2 µm and not greater than 35.64 cm; 2) to sort the sperms of billy goats, the heights of the specimen chambers should be not less than 59.39 µm and not greater than 16.00 cm; 3) to sort the sperms of bulls, the heights of the specimen chambers should be not less than 53.53 µm and not greater than 13.68 cm; 4) to sort the sperms of boars, the heights of the specimen chambers should be not less than 54.6 µm and not greater than 8.06 cm; and 5) to sort the sperms of roosters, the heights of the specimen chambers should be not less than 99.36 µm and not greater than 7.65 cm.

The effects and advantages of the present disclosure have been stated above and are summarized as follows:
1. According to the present disclosure, the first sleeve 42 of the first sorter 40 can be placed into, and positioned in, the receiving space 33 to squeeze the air already in the first specimen chamber 211 out of the first specimen chamber 211 or into the first passage gap 212. This enhances sperm sorting quality by allowing the motile sperms 110 in the semen specimen 100 that have high motility to swim upward through the first sorting filter 44 naturally. Experimental results show that the motile sperms 110 obtained according to the disclosure have extremely high total motility and forward motility.
2. According to the present disclosure, the height adjustment unit 9 or 9' provided between the base 3 and the first sorter 40 makes it possible to adjust the volume of the first specimen chamber 211 and the size of the first passage gap 212.
3. According to the present disclosure, the washer 95 provided between the top surface 322 of the base 3 and the annular bottom surface 432 of the first protruding ring 43 of the first sorter 40 helps increase the volume of the first specimen chamber 211 and the size of the first passage gap 212.
4. According to the present disclosure, the operation method of superposing the first sorter 40 or the second sorter 50 on the base 3 not only helps enhance sperm sorting quality and simplify the sperm sorting process, but also reduces the chance of incorrect operation.
5. According to the present disclosure, the semen specimen 100 can be added, in drops, into the receiving space 33 through the opening 331 of the base 3 by a pipette instead of a syringe to facilitate operation.
6. According to the present disclosure, the first sorter 40 can be taken out of the base 3 upon completion of a sorting operation and then directly placed on the stage of a microscope in order to analyze the motile sperms 110 obtained. The embodiments described herein, therefore, provides greater ease of operation than the prior art.
7. According to the present disclosure, the second sorter 50 can be taken out of the first sorter 40 upon completion of a sorting operation and then directly placed on the stage of a microscope in order to analyze the motile sperms 111 obtained. The embodiments described herein, therefore, provides greater ease of operation than the prior art.
8. According to the present disclosure, the sorting ring 155 provided on the third mounting member 152 of the third sorter 151 allows an operator to press the needle of a liquid taking device against the annular top surface 1551 so that the first sorting medium 120 and the motile sperms 110 can be drawn without fear that the third sorting filter 158 might be punctured.

The embodiments described above are only some feasible ones of the present disclosure and should not be construed as restrictive of the scope of the disclosure. Any simple equivalent change or modification based on the appended claims and the contents of this specification shall fall within the scope of the disclosure.

## Claims

1. A sperm sorting device comprising:
a base comprising:
a bottom; and
an intermediate ring that connects to an external edge of the bottom via an inner wall surface,
wherein the bottom and the inner wall surface together form a specimen space configured to receive a semen specimen;
a sorting unit comprising:
a sorting ring situated at the bottom of the sorting unit and including a central hole for a sorting filter that spans the central hole and filters the semen specimen, wherein a side of the sorting ring facing the sorting filter is beveled at an angle that increases a permeable surface of the sorting filter; and
a protruding ring on a periphery of the sorting unit, the protruding ring connecting to an outer portion of the sorting ring via an inner sleeve surface, wherein the protruding ring is structurally adapted to sit on top of the base.

2. The sperm sorting device of claim 1, wherein the sorting ring further comprises:
a position-limiting groove indented below a top plane of the sorting ring.

3. The sperm sorting device of claim 2, wherein the position-limiting groove extends inward from a bottom plane of the sorting ring to the top plane of the sorting ring.

4. The sperm sorting device of claim 2, wherein a bottom portion of the position-limiting groove contacts the sorting filter.

5. The sperm sorting device of claim 2, wherein an inside of the position-limiting groove opens to the central hole and a bottom plane of the position-limiting groove contacts the sorting filter.

6. The sperm sorting device of any one of claims 1 to 5, wherein an outer bottom portion of the sorting ring is vertically displaced by the sorting filter from a bottom of the intermediate ring when the sorting unit sits on top of the base.

7. The sperm sorting device of any one of claims 1 to 6, further comprising:
a second sorting unit comprising:
a liquid drawing ring situated at the bottom of the second sorting unit and including a second central hole for a second sorting filter that spans the second central hole and filters the semen specimen; and
a second protruding ring on a periphery of the second sorting unit, the second protruding ring connecting to an outer portion of the liquid drawing ring via a second inner sleeve surface, wherein the second protruding ring is structurally adapted to sit on top of the sorting unit.

8. The sperm sorting device of claim 7, wherein the liquid drawing ring of the second sorting unit includes a position-limiting groove indented below a top plane of the liquid drawing ring.

9. The sperm sorting device of claim 8, wherein the sorting ring of the sorting unit includes a set of vent holes that extend through the sorting ring.

10. The sperm sorting device of any one of claims 1 to 9, wherein the sorting filter includes:
a permeable portion that bulges vertically; and
a fixed portion surrounding the permeable portion, wherein the fixed portion contacts the sorting ring.

11. The sperm sorting device of any one of claims 1 to 10, wherein an outer bottom portion of the sorting drawing ring is vertically displaced by the sorting filter from a bottom of the intermediate ring when the sorting unit sits on top of the base.

12. The sperm sorting device of any one of claims 1 to 11, wherein the inner wall surface is angled towards a center of the base.

13. The sperm sorting device of any one of claims 1 to 12, wherein a diameter of the intermediate ring is larger than a diameter of the bottom.

14. A sperm sorting device comprising:
a base comprising:
a bottom; and
an intermediate ring that connects to an external edge of the bottom via an inner wall surface,
a first sorting unit comprising:
a sorting ring including a first central hole for a first sorting filter that spans the first central hole and filters the semen specimen,
wherein a side of the sorting ring facing the sorting filter is beveled at an angle, and
a second sorting unit comprising:
a liquid drawing ring including a position-limiting groove and a second central hole for a second sorting filter that spans the second central hole and filters the semen specimen.

15. A sperm sorting device comprising:
a base comprising:
a bottom; and
a surrounding wall including a coaxial cylinder connecting at a top portion to the bottom via an inner wall surface;
wherein the bottom and the inner wall surface together form a specimen space configured to receive a semen specimen;
a sorting unit configured to sit on the base, comprising:
a sleeve surrounding a same axis as the coaxial cylinder and configured to be placed in the coaxial cylinder, the sleeve having an inner sleeve surface that extends towards a bottom of the sorting unit; and
a sorting filter fixed to the sleeve, the sorting filter and the inner sleeve surface of the sleeve jointly defining a sorting chamber with an open top end;
wherein, the sorting filter contacts the specimen space when the sleeve of the sorting unit is placed in the coaxial cylinder of the surrounding wall of the base.
